# EUROPÄISCHE PATENTSCHRIFT

(11) **EP 3 911 388 B1**
(45) Veröffentlichungstag und Bekanntmachung des Hinweises auf die Patenterteilung: **23.08.2023**
(21) Anmeldenummer: 20700883.0
(22) Anmeldetag: 14.01.2020
(51) Int. Cl.: A61M 15/00

(54) **VORRICHTUNG ZUM INHALIEREN PULVERFÖRMIGER SUBSTANZEN**
DEVICE FOR INHALING POWDER-TYPE SUBSTANCES
DISPOSITIF POUR L'INHALATION DE SUBSTANCES PULVÉRULENTES

(30) Priorität: 14.01.2019 DE 102019100834; 13.01.2020 DE 102020100551
(43) Veröffentlichungstag der Anmeldung: 24.11.2021
(73) Patentinhaber: Von Schuckmann, Alfred, 47627 Kevelaer (DE)
(72) Erfinder: Von Schuckmann, Alfred, 47627 Kevelaer (DE)
(74) Vertreter: Müller, Enno
(86) Internationale Anmeldenummer: PCT/EP2020/050814
(87) Internationale Veröffentlichungsnummer: WO 2020/148281

(56) Entgegenhaltungen:
- EP-A1- 1 992 376
- EP-A1- 3 111 978
- WO-A1-2018/195086
- GB-A- 2 407 042
- US-A1- 2010 078 021
- US-A1- 2010 294 278
- US-A1- 2011 094 510
- US-A1- 2018 214 645

## Beschreibung

### Gebiet der Technik

Die Erfindung betrifft eine Vorrichtung zum Inhalieren pulverförmiger Substanzen nach den Merkmalen des Oberbegriffes des Anspruches 1.

### Stand der Technik

Vorrichtungen der in Rede stehenden Art dienen zur Inhalation, insbesondere zur Inhalation pulverförmiger Substanzen. Die zu inhalierende Substanz ist portioniert in Substanzbehältern bereitgestellt, zur Ausgabe über eine im Zuge eines Einatmens durch den Benutzer aufgebaute Luftströmung. Der hierzu in eine Ausgabe-Bereitschaftsstellung verbrachte Substanzbehälter wird bevorzugt vor Aufbau der Luftströmung durch eine Einstecheinrichtung geöffnet. Über die sich hierbei ergebende Öffnung wird die in dem Behälter portioniert bevorratete Substanz ausgeräumt und über den Austragskanal zum Mundstück transportiert.

Bekannt sind weiter Vorrichtungen, bei welchen eine Mehrzahl von Substanzbehältern in einer Verlagerungsrichtung der Substanzbehälter hintereinander angeordnet in der Vorrichtung vorgesehen sind. Dabei können die Substanzbehälter untereinander verbunden sein, beispielsweise zufolge Ausgestaltung der Substanzbehälter in Art einer Blisterpackung oder eines Blisterstreifens.

Die Substanzbehälter werden mit jeder Betätigung der Vorrichtung sukzessive in die Entleerungsstellung verlagert.

Weiter ist beispielsweise aus der WO 2005/049121 A1 (US 2007/0131 225 A1) eine Vorrichtung der in Rede stehenden Art bekannt, in welcher eine Vielzahl von Substanzbehältern aufgenommen sind, die zur Entleerung sukzessive in eine Entleerungsposition verbracht werden.

Die WO 2003/061 743 A1 (US 8 511 304 B2) offenbart eine Vorrichtung, in welcher die Substanzbehälter kettenartig in Art eines Streifen-Blisters miteinander verbunden in der Vorrichtung vorgesehen sind, wobei die Substanzbehälter so ausgebildet sind, dass diese zwei gegebenenfalls, wie auch bevorzugt, unterschiedliche Substanzen in vorgegebenen Dosierungen bevorraten. Diese beiden Substanzen eines Substanzbehälters sind in bis zu der Entleerung voneinander getrennten Teilbereichen des Substanzbehälters aufgenommen.

Aus der WO 2008/195086 A1 ist eine Vorrichtung zum Inhalieren pulverförmiger Substanzen bekannt, bei der zwei gegenüberliegende Einstechvorrichtungen, die jeweils von Hand betätigbar sind, ausgebildet sind. Aus der EP 3 111 978 A1 ist weiter eine entsprechende Vorrichtung bekannt, bei welcher gleichfalls die Einstecheinrichtungen von Hand zu betätigen sind. Aus der US 2018/2 146 451 A1 ist eine entsprechende Vorrichtung bekannt, bei welcher eine Einstecheinrichtung von Hand zu bewegen ist, mittels derer dann im weiteren auch der Substanzbehälter auf eine zweite, feststehende Einstecheinrichtung zu schieben ist.

Aus der US 2010/0 294 278 A1 ist eine entsprechende Vorrichtung bekannt, bei welcher eine Zählung von darin aufgenommenen Blisterpackungen möglich ist. Darüber hinaus ist es hieraus bekannt, eine Nockenscheibe vorzusehen, mit welcher über Verbindungselemente auf eine Vibrationseinrichtung und eine Einstecheinrichtung einwirkbar ist.

Aus der US 2011/0094510 A1 ist eine Vorrichtung zum Inhalieren bekannt, bei welcher die Verschlusskappe in ihre volle Öffnungsstellung verschwenkt werden muss, um das Einstechen des Einstechmittels zu erreichen. Während des Inhalierens, bei vollständig geöffneter Kappe, ist das Einstechmittel in einen Blister, in dem sich das zu inhalierende Pulver befindet, eingefahren.

Aus der GB 2 407 042 A ist eine Vorrichtung zum Inhalieren bekannt, bei welcher zunächst die Verschlusskappe in die Offenstellung verschwenkt werden muss, bevor eine Einstecheinrichtung, die mit dem Mundstück verbunden ist, in eine Inhalationsstellung verschwenkt werden kann. In der Inhalationsstellung befinden sich die Einstecheinrichtungen auch in der dort als Blister ausgebildeten Verpackung des zu inhalierenden Pulvers.

Ausgehend von einem Stand der Technik wie er etwa aus der US 2011/0094510 A1 bekannt ist, beschäftigt sich die Erfindung mit der Aufgabenstellung, eine Vorrichtung zum Inhalieren pulverförmiger Substanzen anzugeben, die insbesondere vorteilhaft betriebssicher handhabbar ist.

Diese Aufgabe ist beim Gegenstand des Anspruches 1 gelöst, wobei darauf abgestellt ist, dass die Vorrichtung eine verschwenkbare Verschlusskappe aufweist und dass mit Verschwenken der Verschlusskappe zugleich auf beide Einstecheinrichtungen eingewirkt wird und dass am Ende einer Verschwenkbewegung der Verschlusskappe aus einer Vorrichtungs-Verschlussstellung in eine Vorrichtungs-Offenstellung jede Einstecheinrichtung wieder aus dem Substanzbehälter herausgefahren ist.

### Zusammenfassung der Erfindung

Durch die Anordnung von zwei, bevorzugt auch hinsichtlich der Halteteile voneinander gesonderten Einstecheinrichtungen ist der Substanzbehälter in zwei, gegebenenfalls zueinander distanzierten Bereichen öffenbar. Die hierdurch an dem Substanzbehälter erreichbaren Öffnungen können zueinander distanziert ausgebildet werden. Die Bereiche können aus unterschiedlichen Richtungen erreichbar ausgebildet sein.

Die Einstechrichtungen können auch in unterschiedlichen Ebenen gegeben sein, so dass sie nicht notwendig - wenn auch linear, beispielsweise entgegengesetzt, verlaufend - miteinander fluchten. Jede Einstecheinrichtung kann ein gesondertes Halteteil aufweisen, an welchem jeweils ein Einstechmittel angeordnet sein kann. Die Einstecheinrichtungen, insbesondere hinsichtlich der Halteteile und/oder der Einstechmittel können unterschiedlich gestaltet sein, so dass beispielsweise unterschiedliche Öffnungsquerschnitte in dem Substanzbehälter erreicht werden können.

Die Einstechrichtungen der beiden Einstecheinrichtungen können im Wesentlichen aufeinander zu gerichtet sein, darüber hinaus aber auch gegebenenfalls mit Bezug auf eine Draufsicht oder in einem Querschnitt, in welchem sich die Einstechrichtungen jeweils linienartig darstellen, einen spitzen Winkel von beispielsweise wenigen Winl<elgeraden, wie weiter beispielsweise 5 oder 10 Grad, bis hin zu etwa 90 Grad, darüber hinaus aber auch gegebenenfalls einen stumpfen Winkel bis hin zu etwa 180 Grad zueinander einschließend. Dabei können sich die Linien der Einsteckrichtungen innerhalb des Substanzbehälters treffen, alternativ aber auch im Wesentlichen außerhalb desselben oder eben auch höhenmäßig versetzt, ohne sich zu treffen, das heißt windschief, verlaufen.

Zufolge dieser Ausgestaltung kann durch die zwei Öffnungen des Substanzbehälters die bevorratete Substanz verbessert ausgetragen werden. Es ergibt sich eine günstige Räumung des Behälters. Darüber hinaus kann zufolge der vorgeschlagenen Ausgestaltung auch ein Substanzbehälter in günstiger Weise geöffnet werden, der mehrere, beispielsweise zwei Kavitäten mit Substanz aufweist. Die beiden Kavitäten können bevorzugt unterschiedliche pulverförmige Medikamente aufweisen, die weiter bevorzugt erst bei Durchführung der Inhalation im Austragskanal und/ oder im Mundstück vereint werden können. Entsprechend ist jede Kavität des Substanzbehälters in der Entleerungsstellung zu öffnen. Dies wird durch die zwei voneinander gesonderten Einstecheinrichtungen bewirkt.

Mit den zwei voneinander gesonderten Einstechbereichen kann gesondert zur Öffnung des Substanzbehälters auf diesen eingewirkt werden. Sie können so gesonderte Öffnungen oder, wenn ein Einstechbereich in Einzelheit mit mehreren Einstechvorsprüngen, siehe auch die nachstehend beschriebenen mehreren Einstechspitzen, ausgebildet ist, gesonderte Öffnungsbereiche in dem Substanzbehälter ausbilden. Die Einstechbereiche können bezüglich ihrer Einstechgeometrien unterschiedlich gestaltet sein, wenngleich eine gleiche, insbesondere klappsymmetrische Ausgestaltung der Geometrien der Einstechbereiche bevorzugt ist.

Dabei kann zwischen den Einstechbereichen ein sich in Transportrichtung der Substanzbehälter ersteckender Freiraum gegeben sein. Entsprechend kann sich eine Beabstandung der Einstechbereiche quer zu der Transportrichtung der Substanzbehälter ergeben, in welchem sich daraus ergebenden Beanstandungsbereich keine Perforation bzw. kein Öffnen des Substanzbehälters bevorzugt erfolgt. Dies kann sich an dem geöffneten Substanzbehälter beispielsweise in Form eines zwischen zwei Öffnungsbereichen sich einstellenden Stegs oder Brückenabschnitts widerspiegeln. So können sich gemäß einer möglichen Ausgestaltung an dem Substanzbehälter zugeordnet einem Einstechmittel eine definierte Einströmöffnung und eine definierte Ausströmöffnung ergeben, welche Öffnungen zueinander beispielsweise durch einen Steg distanziert sind.

In einer weiter möglichen Ausgestaltung kann ein Einstechbereich eine zusammenhängende, gegebenenfalls einteilige Einstechspitze aufweisen. Alternativ können aber auch an einem Einstechbereich zugeordnet dem Substanzbehälter zwei oder mehr Einstechspitzen ausgebildet sein, so dass sich nach einem Einstechvorgang eine mehrfache punktartige Perforation ergeben kann. So können weiter beispielweise drei oder mehr, weiter beispielsweise vier oder fünf bis hin zu zehn Einstechspitzen je Einstechbereich vorgesehen sein.

Eine Einstechspitze kann beispielsweise dornartig ausgebildet sein, mit einem an einem zylindrischen Bereich anschließenden Spitzenbereich. Das größte Durchmessermaß des Spitzenbereiches kann dem Durchmessermaß des zylindrischen Bereichs entsprechen, der wiederum mit seinem Durchmessermaß bevorzugt das freie Öffnungsmaß einer durch die Einstechspitze verursachten Öffnung in dem Substanzbehälter bestimmt.

Die Verlagerung der Einstecheinrichtungen, insbesondere der das Einstechmittel jeweils aufweisenden Halteteile, in die Einstechstellung zur Öffnung des Substanzbehälters kann gemäß einer möglichen Ausgestaltung gegen die Kraft einer die Einstecheinrichtung rückstellenden Feder erfolgen. Es kann diesbezüglich eine übliche Metallfeder, in Form einer Druckfeder oder einer Blattfeder vorgesehen sein. Bevorzugt wird diesbezüglich eine kombinierte Ausbildung des Halteteils mit einer Kunststofffeder. So kann das Halteteil bei Ausbildung desselben als beispielsweise Kunststoffspitzteil einstückig und bevorzugt materialeinheitlich mit der Kunststofffeder ausgeformt sein.

Dabei kann die Kunststofffeder zwei entgegengesetzt gerichtete Federarme ausbilden. Über diese Federarme kann das Halteteil sich an einem relativ zu dem Halteteil feststehenden Vorrichtungsabschnitt, beispielsweise Gehäuseabschnitt, abstützen.

In einer bevorzugt unbelasteten Grundstellung einer jeden Einstecheinrichtung, aus welcher Grundstellung heraus eine Verlagerung insbesondere des Halteteils mit dem hieran angeordneten Einstechmittel zum Öffnen des Substanzbehälters erfolgt, kann die auf das Halteteil einwirkende Kunststofffeder im Wesentlichen entspannt sein, so dass sich eine auf das Halteteil einwirkende Rückstellkraft der Kunststofffeder einstellen kann, die gleich oder annähernd gleich Null sein kann.

Die Federarme der Kunststofffeder können zugleich zur Führung des Halteteils bei einem Einstechvorgang dienen. Hierzu können die Federarme mit bevorzugt relativ zu den Halteteilen feststehenden Abschnitten der Vorrichtung zusammenwirken. Hierüber kann ein reproduzierbarer Einstechvorgang durchgeführt werden.

Ausgehend von ihren freien Enden können die Federarme eine Führungsausnehmung aufweisen, die mit einem gehäusefesten Zapfen zusammenwirkt. Die Führungsausnehmung kann beispielsweise schlitzartig gestaltet sein, in welchen der gehäusefeste Zapfen eintaucht. Auch kann über diesen Zapfen eine Festlegung der Federarme und hierüber der gesamten Einstecheirichtung beispielsweise an dem Vorrichtungsgehäuse erreicht sein.

Die Federarme können in einer Seitenansicht, in der sich eine Einstechrichtung linienartig darstellt und beide Federarme sich in ihrer Längserstreckung abbilden, konkav gebildet sein, mit dem Halteteil zugeordnet einer Längsmitte der zusammengefassten Federarme. Die sich insgesamt im Wesentlichen aus den beiden Federarmen zusammensetzende Kunststofffeder kann sich entsprechend brücken- oder gewölbeartig erstrecken, gegebenenfalls mit jeweils endseitigen, fundamentartigen Befestigungs- und/oder Führungsabschnitten, wobei im Wesentlichen im Gewölbezenit der Kunststofffeder zwischen den beiden Federarmen das Halteteil angeordnet sein kann. Das Halteteil mit dessen Einstechmittel kann hierbei, wie auch bevorzugt, in den durch die konkav angeordneten Federarme gebildeten Gewölberaum eintauchen.

Es kann so bei einer entsprechenden Verlagerung des Halteteils mit dem Einstechmittel in Richtung auf den Substanzbehälter eine gleichmäßige, beidseitige Führung des Halteteils über die Federarme gegeben sein, wobei diese Verlagerung entgegen der sich aufbauenden Rückstellkraft der Federarme erfolgen kann. Mit Fortfall einer diese Verlagerung des Halteteils in die Einstechstellung bewirkenden Belastung kann das Halteteil mit dem Einstechmittel und somit die gesamte Einstecheinrichtung über die Rückstellkraft der Feder in die Grundstellung zurückverlagert werden.

In einer bevorzugten Ausgestaltung sind beide Einstecheinrichtungen gleichgestaltet.

Auch können zwei Austragskanäle vorgesehen sein, beispielsweise jeweils zugeordnet einer Einstecheinrichtung und/oder jeweils zugeordnet einer Kavität des Substanzbehälters, bei Ausbildung von beispielsweise zwei Kavitäten je Substanzbehälter. Diese Austragskanäle können in Strömungsrichtung betrachtet vor oder auch unmittelbar im Übergang zu dem Mundstück zusammengeführt sein. Somit können zwei Teilströme mit gegebenenfalls unterschiedlicher Substanz aus der Kavität des jeweiligen Substanzbehälters entstehen, die im Bereich des Mundstückes zusammentreffen. Hierbei kann weiter in dem Mundstück oder in dem jeweiligen Teil-Austragskanal eine Umlenkschikane oder dergleichen vorgesehen sein, um eine bessere Verteilung und/oder Zerbröselung von Substanz zu erreichen. Auch kann innerhalb des Mundstücckanals eine solche Verwirbelungseinrichtung oder dergleichen vorgesehen sein.

In weiterer Ausgestaltung können - bevorzugt zugeordnet einem Einstechbereich - zwei Ansaugkanäle vorgesehen sein. Dabei wird im Zuge beispielsweise eines Inhalationsvorganges über beide Ansaugkanäle Luft, insbesondere Außenluft angesaugt. Die Ansaugkanäle können bezüglich ihrer Länge in Strömungsrichtung und bevorzugt auch bezüglich ihrer quer zur Strömungsrichtung betrachteten Querschnittsgestaltung zumindest annähernd gleich ausgebildet sein. Beide Ansaugkanäle führen bevorzugt unmittelbar zu dem in der Entleerungsposition befindlichen Substanzbehälter.

Einer oder beide Ansaugkanäle können in einer Strömungsrichtung vor dem Substanzbehälter über einen Bypass mit dem Austragskanal verbunden sein. Der Bypass bietet dabei einen Verbindungskanal der beiden Ansaugkanäle untereinander an, der bevorzugt unmittelbar in den Austragskanal, entsprechend unter Umgehung des bevorzugt geöffneten Substanzbehälters, mündet. Ist über den Strömungsweg durch den Substanzbehälter, beispielsweise aufgrund der gegebenen Kanal- und Behälterquerschnitte, nur eine beschränkte Luft-Durchsatzmenge führbar, kann im Zuge einer üblichen Beaufschlagung durch Ansaugen über die Ansaugkanäle ein (überschüssiger) Anteil der Ansaugluft durch den Bypass geleitet werden.

Auch kann in der Vorrichtung eine Mehrzahl von Substanzbehältern gleichzeitig aufnehmbar sein. Hierbei kann es sich um eine in der Vorrichtung bevorzugt nicht unterbrochene Reihe an Substanzbehältern handeln, wobei die Substanzbehälter schrittweise für jeden einzelnen Inhalationsvorgang in eine Entleerungsstellung verbracht werden können. Die Substanzbehälter können untereinander unverbunden sein und in einer vorrichtungsfesten Führungseinrichtung zur unmittelbaren Anlage aneinander aufgenommen sein, wobei die Bewegung der Substanzbehälter durch einen sich unter den Substanzbehältern fortpflanzenden Anlagedruck gegeben sein kann.

Diese Bewegung der Substanzbehälter kann über eine von außen für den Benutzer zugängliche Antriebswelle erreichbar sein.

Eine solche Antriebswelle kann ein antreibbares Antriebsritzel für das Zählwerk aufweisen, welches Antriebsritzel darüber hinaus über ein Übertragungszahnrad auf ein Zählrad einwirken kann. Das Zählrad kann Symbole, gegebenenfalls, wie auch bevorzugt, Zahlen tragen, die beispielsweise durch ein Sichtfenster in dem Vorrichtungsgehäuse von außen erkennbar sind.

Antriebsritzel, Übertragungszahnrad und Zählrad können getriebeartig zusammenwirken, entsprechend kann sich eine Unter- oder auch Übersetzung von der Antriebswelle auf das Zählrad ergeben.

Für eine ordnungsgemäße Anordnung insbesondere des Zählrades, weiter insbesondere eine ordnungsgemäße Ausrichtung des Zählrades in einer Grundposition, in welcher beispielsweise die Anzahl der entleerten Substanzbehälter Null ist oder die Anzahl der nichtentleerten Substanzbehälter der Maximalanzahl an Substanzbehältern in der Vorrichtung entspricht, kann vorgesehen sein, dass für das Übertragungszahnrad und das Zählrad Ausrichtungsausformungen an dem Gehäuse und/oder zwischen dem Übertragungszahnrad und dem Zählrad vorgesehen sind, die eine Montage des Übertragungszahnrades und des Zählrades (nur) in einer vorgegebenen Winkelausrichtung ermöglichen. Es sind so Montagehilfen zum Ein- beziehungsweise Ansetzen des Übertragungszahnrades und des Zählrades gegeben. Die Einbauposition ist hierdurch vorgegeben. Den gehäuseseitigen Ausrichtungsausformungen können hierzu an dem Übertragungszahnrad und/oder an dem Zählrad ausgebildete Gegenausformungen zuordbar sein. So kann das Übertragungszahnrad gemäß einer bevorzugten Ausgestaltung nur in einer vorgegebenen Drehstellung relativ zu der Antriebswelle in das Gehäuse eingesetzt werden und das Zählrad in nur einer relativen Drehposition zu dem Übertragungszahnrad und/oder der Antriebswelle. Aus diesen Drehpositionen heraus wird das Zählrad über die Antriebswelle und das Übertragungszahnrad bei Betätigung der Vorrichtung und bevorzugter Inhalation um einen Zählschritt weiter bewegt.

Die beim Einsetzen vorgegebene Drehstellung ist bevorzugt jedoch noch nicht eine Benutzungs-Ausgangsstellung. Diese kann gegebenenfalls erst erreicht werden, nachdem die ansonsten betriebsbereite Vorrichtung mit den Substanzbehältern bestückt wurde. Im Zuge dieser Bestückung wird beispielsweise mit jedem Einführen eines Substanzbehälters das Zählrad um einen Schritt weiter in Richtung auf dessen Benutzungs-Ausgangsstellung gedreht, die mit Einsetzen des letzten Substanzbehälters in die Vorrichtung erst erreicht sein kann.

Die Vorrichtung kann eine verschwenkbare Verschlusskappe aufweisen. Über diese kann beispielsweise das Mundstück in der Nichtbenutzungsstellung der Vorrichtung überdeckt sein. Die Verschlusskappe kann dabei um eine geometrische Schwenkachse aus einer Vorrichtungs-Verschlussstellung in eine Vorrichtungs-Öffnungsstellung verlagert werden, welche geometrische Schwenkachse der geometrischen Schwenkachse der Antriebswelle entsprechen kann.

Mit Verschwenken der Verschlusskappe kann auf die Antriebswelle eingewirkt werden, über welche Antriebswelle mittelbar oder unmittelbar gegebenenfalls eine Verlagerung eines Substanzbehälters in die Entleerungsstation durchgeführt werden kann. Darüber hinaus kann über die Antriebswelle auch die schrittweise Verlagerung des Zählrades erfolgen.

Auch kann mit Verschwenken der Verschlusskappe in einer möglichen Ausgestaltung zugleich auf beide Einstecheinrichtungen eingewirkt werden. Über die Verschwenkbewegung der Verschlusskappe kann in diesem Zusammenhang ein Einwirkmittel derart die Einstecheinrichtung belasten, dass deren Halteteil mit dem Einstechmittel entgegen der Rückstellkraft der Federarme in die Einstechposition verlagert wird. Bevorzugt werden dabei beide Einstecheinrichtungen in gleicher Weise in Richtung auf den Substanzbehälter verlagert. Dabei kann die Rotationsbewegung der Verschlusskappe in eine im Wesentlichen lineare Bewegung der Einstecheinrichtungen, insbesondere eine lineare Bewegung der Einstechmittel, übertragen werden. Diese lineare Bewegung der Einstechmittel kann dabei im Wesentlichen in Richtung der geometrischen Schwenkachse der Verschlusskappe gegeben sein.

Gemäß einer weiter bevorzugten Ausgestaltung kann am Ende der Verschwenkbewegung der Verschlusskappe aus der Vorrichtungs-Verschlussstellung in die Vorrichtungs-Offenstellung, die darüber hinaus anschlagbegrenzt sein kann, jede Einstecheinrichtung wieder aus dem Substanzbehälter herausgefahren sein. Dies kann erreicht sein zufolge Fortfall der auf die Einstecheinrichtungen wirkenden Belastung über die Verschlusskappe. Die Anordnung und Ausbildung des die jeweilige Einstecheinrichtung belastenden, über die Verschlusskappe bewegten Stellmittels kann hierzu konstruktiv so vorgesehen sein, dass dieses Mittel noch vor Erreichen der Verschlusskappen-Schwenkendstellung die Einstecheinrichtungen verlässt, so dass diese aufgrund der Federrückstellkräfte ihre Einstechmittel aus den Substanzbehälter herausfahren und in Richtung auf ihre Grundstellung sich verlagern.

Diese Verschlusskappen-Schwenkendstellung kann, wie auch bevorzugt, der Inhalations-Bereitschaftsstellung entsprechen. Durch Saugen an dem Mundstück wird eine Luftströmung aufgebaut, die den Substanzbehälter oder eine Mehrzahl von Kavitäten in dem Substanzbehälter räumt. Die ausgetragene Substanz wird über den einen Austragskanal oder über die mehreren Austragskanäle zum Mundstück transportiert und hierüber inhaliert.

### Kurze Beschreibung der Zeichnungen

Nachstehend ist die Erfindung anhand der beigefügten Zeichnung erläutert, die aber lediglich Ausführungsbeispiele darstellt. Ein Teil, das nur bezogen auf eines der Ausführungsbeispiele erläutert ist und bei einem weiteren Ausführungsbeispiel aufgrund der dort herausgestellten Besonderheit nicht durch ein anderes Teil ersetzt ist, ist damit auch für dieses weitere Ausführungsbeispiel als jedenfalls mögliches vorhandenes Teil beschrieben. Die Zeichnung zeigt:
- Fig. 1: eine Vorrichtung zum Inhalieren pulverförmiger Substanzen in perspektivischer Darstellung, betreffend die verschlossene Nichtgebrauchsstellung;
- Fig. 2: die Vorrichtung gemäß Figur 1 in einer weiteren perspektivischen Darstellung;
- Fig. 3: die Herausvergrößerung des Bereiches III vor Verschließen einer gehäuseseitigen Einführöffnung durch ein Verschlussteil;
- Fig. 4: in perspektivischer Einzeldarstellung einen Substanzbehälter für die Vorrichtung;
- Fig. 5: die Draufsicht gemäß Pfeil V in Figur 4;
- Fig. 6: die Ansicht gemäß Pfeil VI in Figur 4 gegen den Substanzbehälter;
- Fig. 7: den Schnitt gemäß der Linie VII-VII in Figur 6;
- Fig. 8: die Herausvergrößerung des Bereiches VIII in Figur 7, bei an dem Substanzbehälter festgelegter Abdeckung;
- Fig. 9: eine der Figur 8 im Wesentlichen entsprechende Detailschnittdarstellung, jedoch betreffend eine Situation vor Festlegung der Abdeckung an dem Substanzbehälter;
- Fig. 10: eine perspektivische Detaildarstellung des Substanzbehälters, partiell geschnitten, betreffend die Situation gemäß Figur 9;

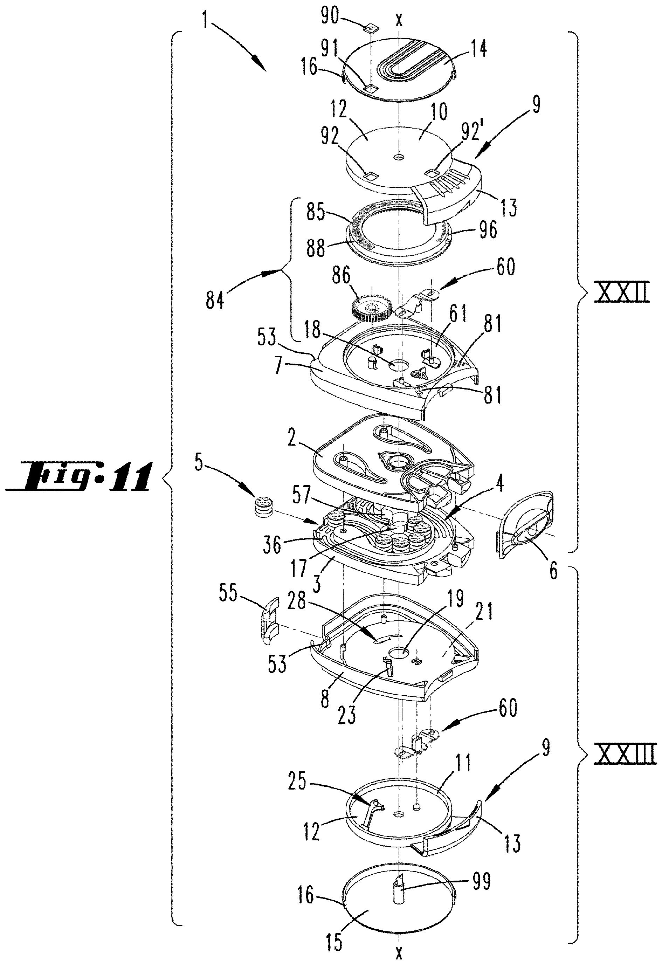

- Fig. 11: eine explosionsperspektivische Darstellung der Vorrichtung;
- Fig. 12: eine vergrößerte explosionsperspektivische Darstellung des Bereiches XII in Figur 11;
- Fig. 13: die vergrößerte explosionsperspektivische Darstellung des Bereiches XIII in Figur 11;
- Fig. 14: die Vorrichtung in einer weiteren explosionsperspektivischen Darstellung;
- Fig. 15: eine vergrößerte explosionsperspektivische Darstellung des Bereiches XV in Figur 14;
- Fig. 16: eine explosionsperspektivische Darstellung des Bereiches XVI in Figur 14;
- Fig. 17: in perspektivischer Darstellung die Anordnung eines Antriebsritzels, eines Antriebselementes und eines drehfest auf einer Antriebswelle angeordneten Betätigungsrades, weiter eines Übertragungszahnrades und eines Zählrades;
- Fig. 18: in perspektivischer Explosionsdarstellung das Zählrad und das Übertragungsrad sowie den das Zählrad und das Übertragungszahnrad aufnehmenden Gehäuseabschnitt;

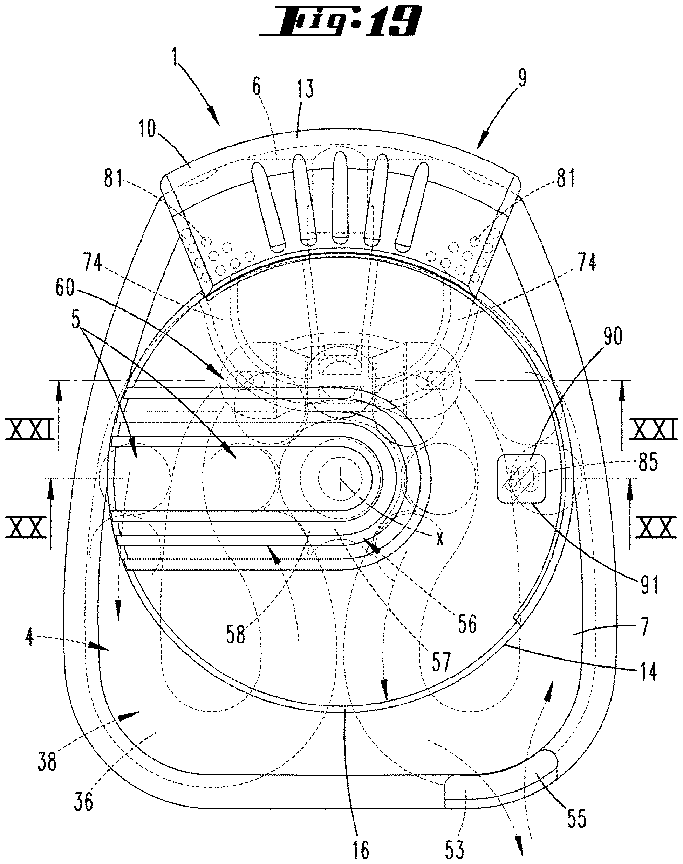

- Fig. 19: die Vorrichtung in Draufsicht, betreffend die verschlossene Nichtgebrauchsstellung;
- Fig. 20: den Schnitt gemäß der Linie XX-XX in Figur 19;

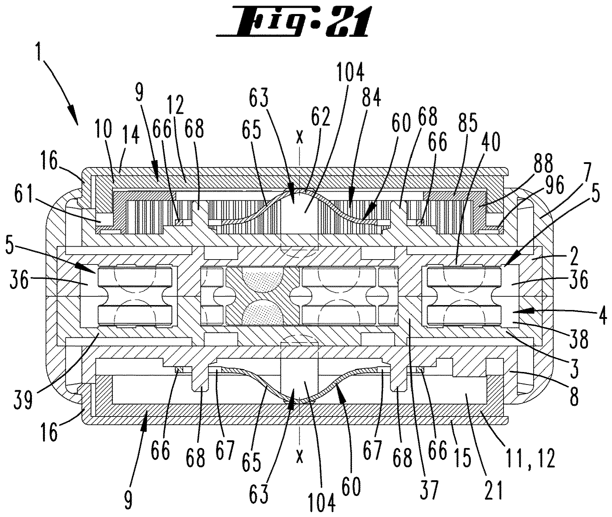

- Fig. 21: den Schnitt gemäß der Linie XXI-XXI in Figur 19;
- Fig. 22: den Schnitt gemäß der Linie XXII-XXII in Figur 20;
- Fig. 23: eine der Figur 22 entsprechende Darstellung, jedoch ohne in der Vorrichtung aufnehmbaren Substanzbehältern;
- Fig. 23a: eine der Figur 23 entsprechende Darstellung, jedoch eine alternative Ausführungsform betreffend;
- Fig. 24: eine weitere der Figur 22 entsprechende Darstellung, betreffend eine Situation im Zuge eines Befüllens der Vorrichtung mit Substanzbehältern;

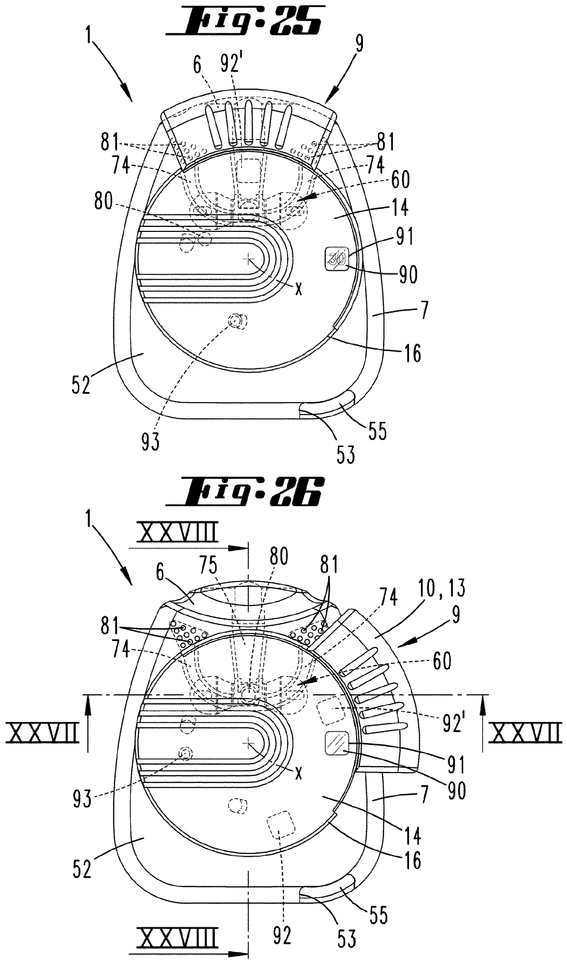

- Fig. 25: eine im Wesentlichen der Figur 19 entsprechende Draufsichtdarstellung der Vorrichtung;
- Fig. 26: eine der Figur 25 entsprechende Darstellung, jedoch im Zuge einer Schwenkbewegung einer Verschlusskappe in Richtung auf eine Öffnungsstellung;

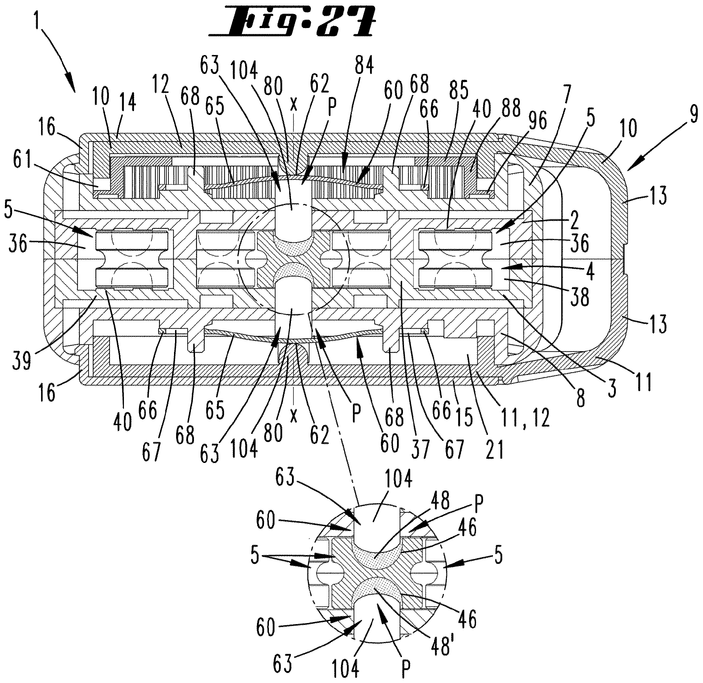

- Fig. 27: den Schnitt gemäß der Linie XXVII-XXVII in Figur 26;

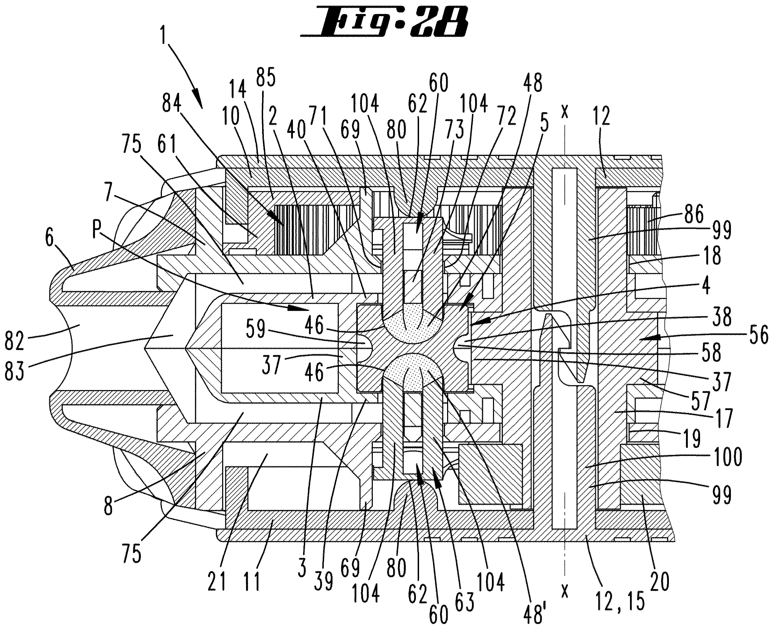

- Fig. 28: den Schnitt gemäß der Linie XXVIII-XXVIII in Figur 26;

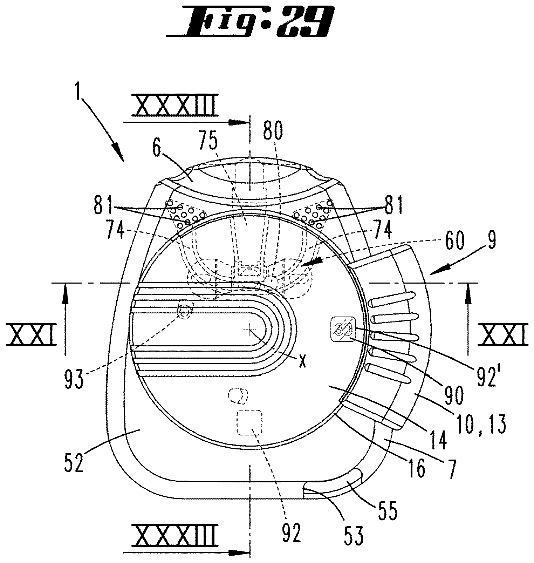

- Fig. 29: eine Folgedarstellung zur Figur 26, die VerschlusskappenOffenstellung betreffend;

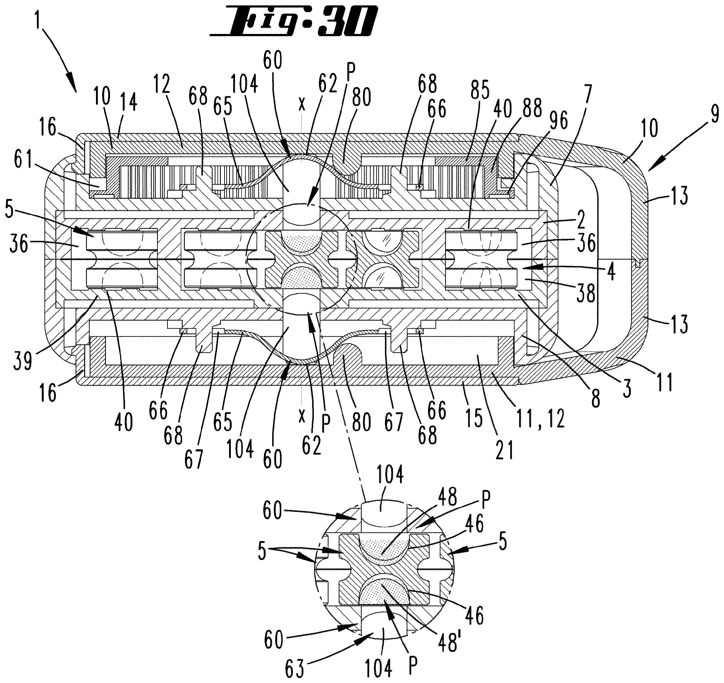

- Fig. 30: den Schnitt gemäß der Linie XXX-XXX in Figur 29;
- Fig. 31: die Vorrichtung in perspektivischer Darstellung, die Verschlusskappen-Offenstellung und somit die InhalationBereitschaftsstellung betreffend;
- Fig. 32: eine im Wesentlichen der Figur 31 entsprechende Darstellung, jedoch partiell aufgebrochen;
- Fig. 32a: eine der Figur 32 entsprechende Darstellung, eine alternative Ausführungsform betreffend;
- Fig. 33: den Schnitt gemäß der Linie XXXIII-XXXIII in Figur 29;

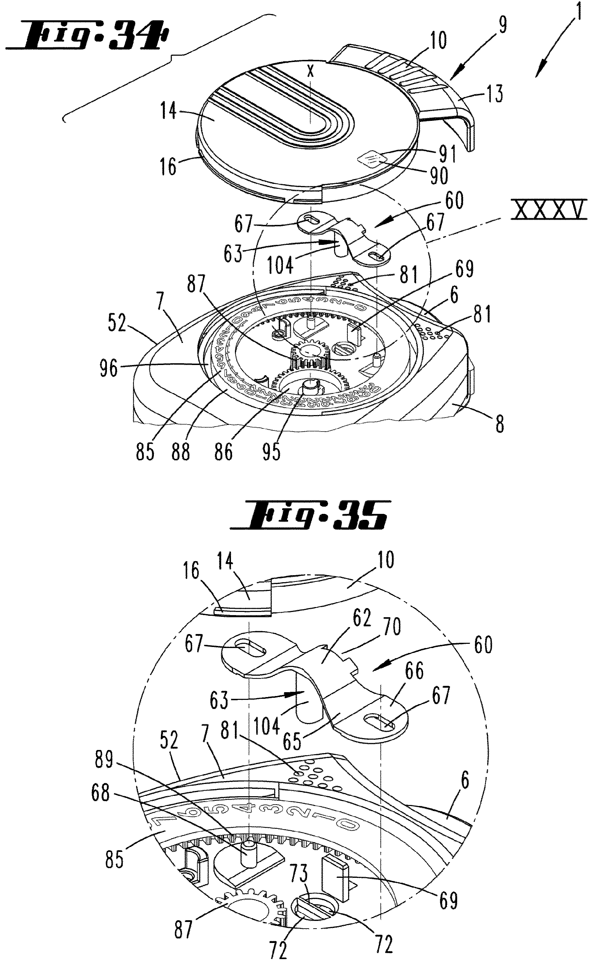

- Fig. 34: eine teilweise explosionsperspektivische Darstellung der Vorrichtung, betreffend den Bereich einer Einstecheinrichtung;
- Fig. 35: die vergrößerte Darstellung des Bereiches XXXV in Figur 34;
- Fig. 36: die Einstecheinrichtung in einer perspektivischen Einzeldarstellung;
- Fig. 36a: die Einstechrichtung in einer zweiten Ausführungsform;
- Fig. 36b: die Herausvergrößerung des Bereichs XXXVIb in Figur 36a;
- Fig. 37: die Einstecheinrichtung in einer Seitenansicht;
- Fig. 38: eine Schnittdarstellung betreffend die Anordnung von zwei Einstecheinrichtungen in der Vorrichtung zum Durchstoßen zweier Abdeckungen eines Substanzbehälters;
- Fig. 38a: eine Schnittdarstellung gemäß Fig. 38, jedoch die Ausführung gemäß Fig. 36a betreffend;
- Fig. 39: den Schnitt gemäß der Linie XXXIX-XXXIX in Figur 37;
- Fig. 39a: die Schnittdarstellung gemäß Figur 39, die zweite Ausführung betreffend;
- Fig. 40: eine der Figur 37 im Wesentlichen entsprechende Seitenansichtsdarstellung, jedoch betreffend die Anordnung von zwei Einstecheinrichtungen;
- Fig. 41: eine perspektivische Schnittdarstellung durch einen Substanzbehälter, bei von einer Einstecheinrichtung gemäß Fig. 36 durchstoßenen Abdeckungen;
- Fig. 41a: eine der Figur 41 entsprechende Darstellung, jedoch bei durch eine Einstechrichtung gemäß Figur 36a durchstoßenen Abdeckungen;
- Fig. 42: eine im Wesentlichen schematische Draufsichtdarstellung auf die Vorrichtung, betreffend das getriebeartige Zusammenwirken des Zählwerks und des Antriebselementes zum Verbringen eines Substanzbehälters in eine Entleerungsposition sowie die Beaufschlagung des Einstechmittels in Abhängigkeit von der Schwenkverlagerung der Verschlusskappe, betreffend die Kappenschließstellung;
- Fig. 43: eine Folgedarstellung zu Figur 42, im Zuge der Verschwenkbewegung der Verschlusskappe in Richtung auf die Offenstellung;

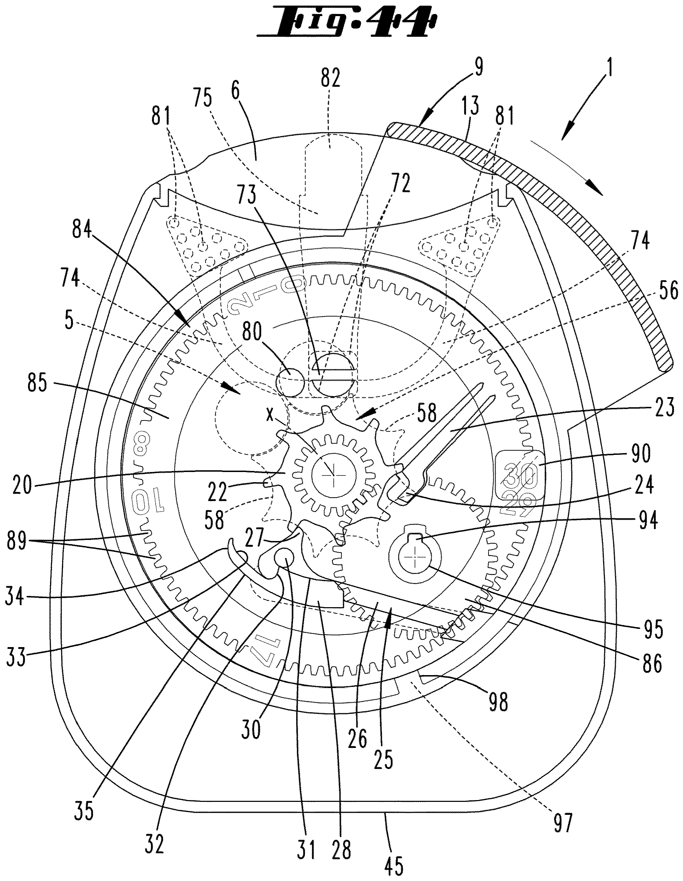

- Fig. 44: eine Folgedarstellung zur Figur 43;

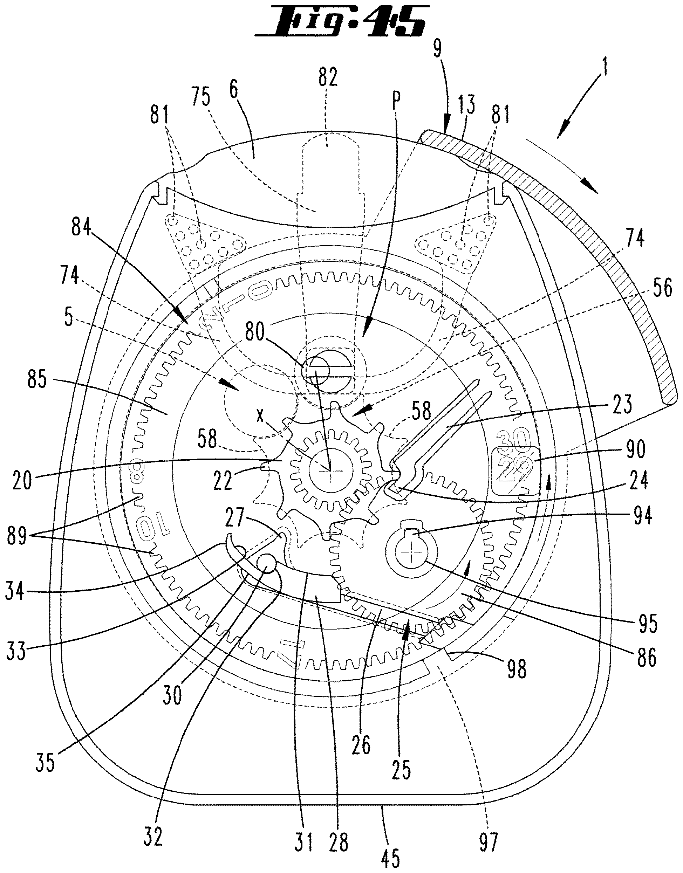

- Fig. 45: eine Folgedarstellung zur Figur 44, betreffend eine Zwischenstellung, in welcher der Substanzbehälter die Entleerungsposition erreicht hat;

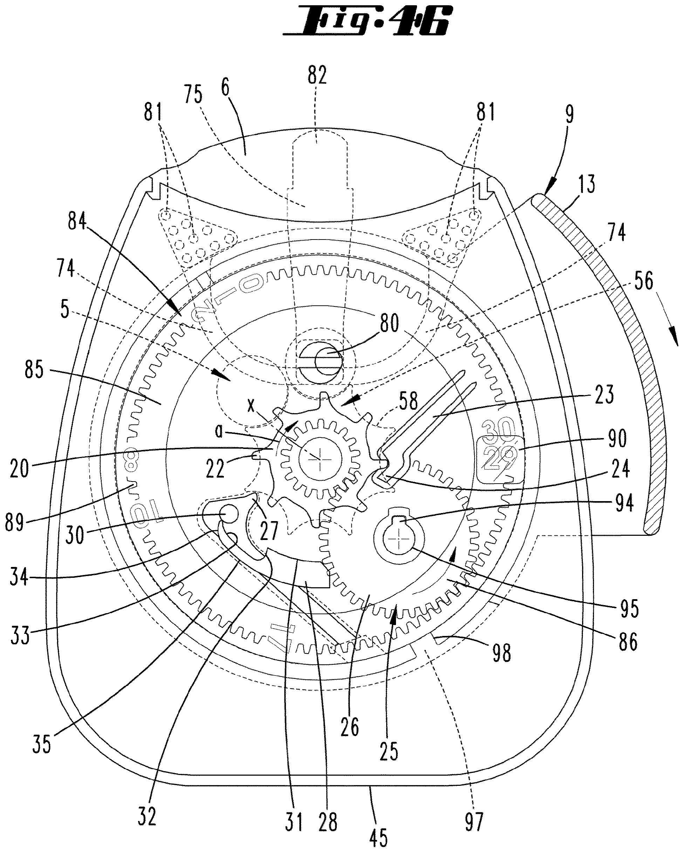

- Fig. 46: eine weitere Folgedarstellung im Zuge der Verschwenkung der Verschlusskappe in die Offenstellung, betreffend die Beaufschlagungssituation des Einstechmittels;
- Fig. 47: eine weitere Folgedarstellung bei Erreichen der Verschlusskappen-Offenstellung;
- Fig. 48: in schematischer Darstellung den Bereich der Luftkanäle gemäß XLVIII in Figur 47;
- Fig. 48a: eine der Figur 48 entsprechende Darstellung, jedoch die Ausführungsform gemäß Figur 32a betreffend;

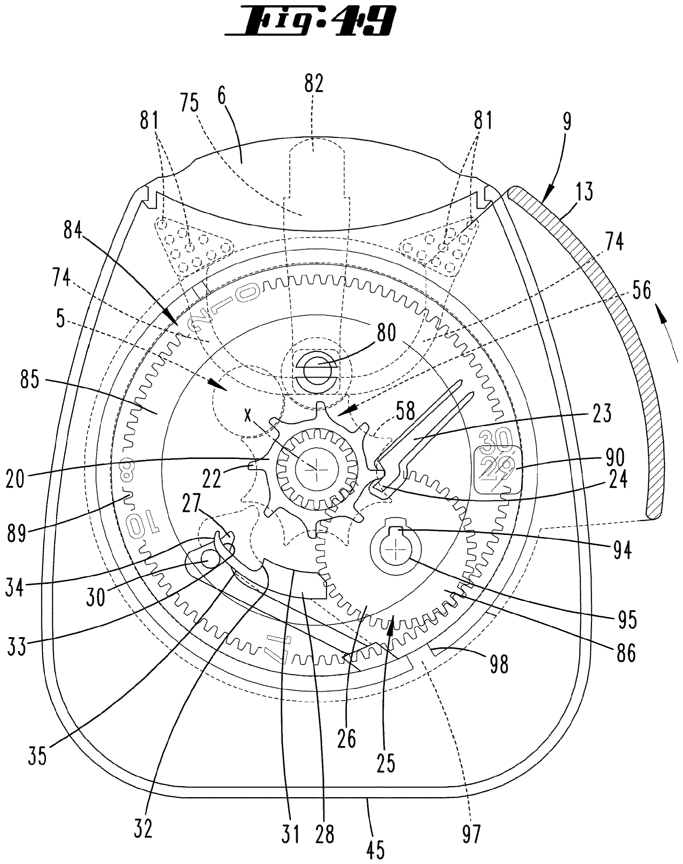

- Fig. 49: eine Zwischenstellung im Zuge der Verschwenkung der Verschlusskappe aus der Offenstellung in Richtung auf die Verschlussstellung;
- Fig. 50: eine schematische Darstellung der Vorrichtung in Draufsicht, zur Erläuterung einer geometrischen Grundkontur der Vorrichtung und deren Abmessungen.

### Beschreibung der Ausführungsformen

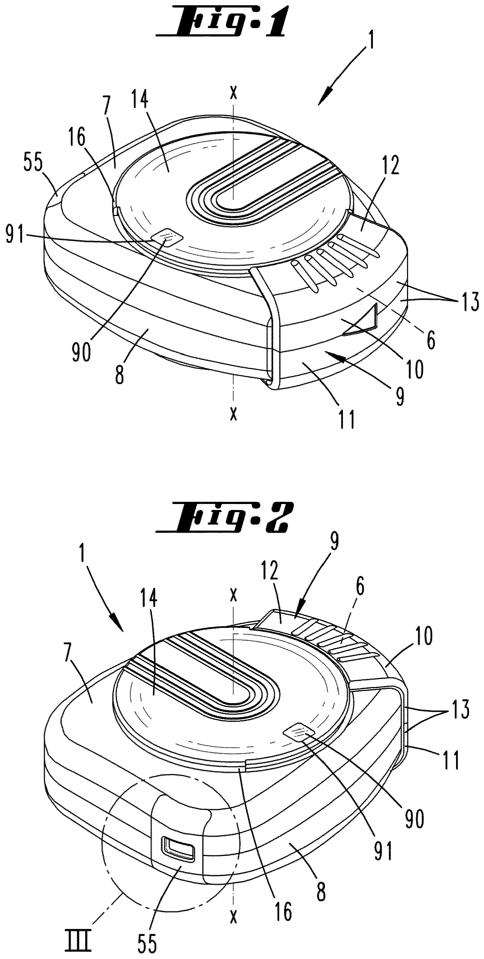

Dargestellt und beschrieben ist, zunächst mit Bezug zu den Figuren 1 und 2, eine Vorrichtung 1 zum Inhalieren pulverförmiger Substanzen 48, 48'. Die Vorrichtung 1 weist bevorzugt ein Mundstück 6 auf, weiter eine Einstecheinrichtung 60 mit Einstechmitteln 63, zur Öffnung eines Substanzbehälters 5, wobei bevorzugt eine Mehrzahl von sukzessiv in eine Entleerungsposition P bewegbare Substanzbehälter 5 vorgesehen sind, die untereinander unverbunden in einer vorrichtungsfesten Führungseinrichtung 4 zur unmittelbaren Anlage aneinander aufgenommen sind. Zur Zählung und Anzeige der durchgeführten oder noch verbleibenden Inhalationsvorgänge ist ein Zählwerk 84 vorgesehen.

Die nachstehend aufgeführten wesentlichen Bestandteile der Vorrichtung 1 können, wie bevorzugt, aus einem Kunststoff, insbesondere Hartkunststoff wie Polypropylen oder Polyethylen bestehen.

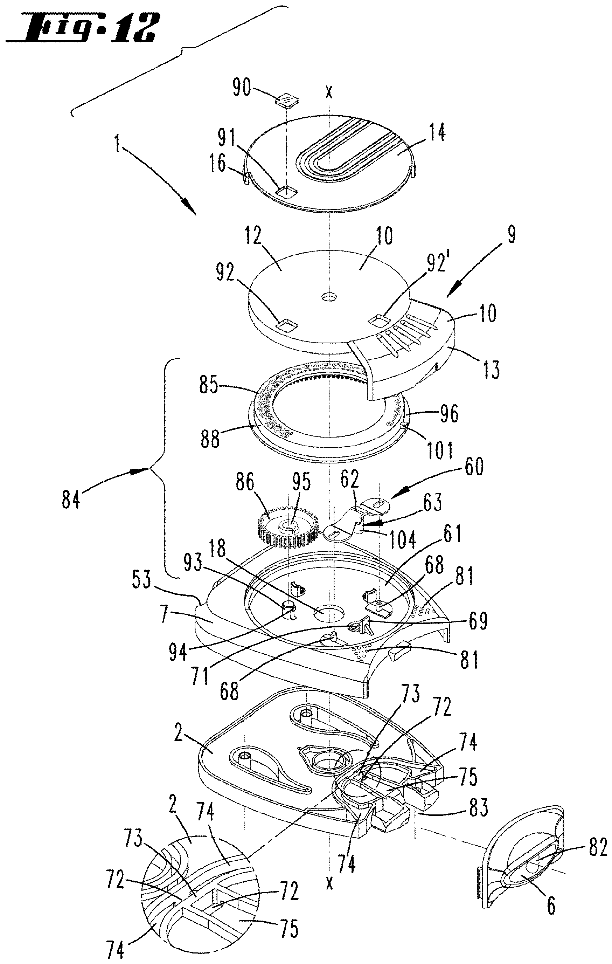

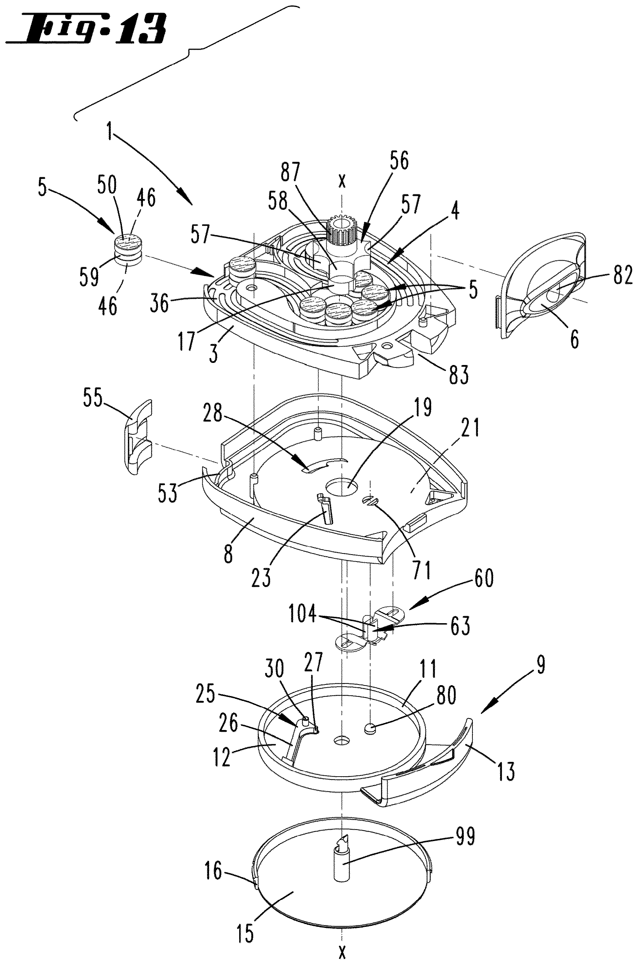

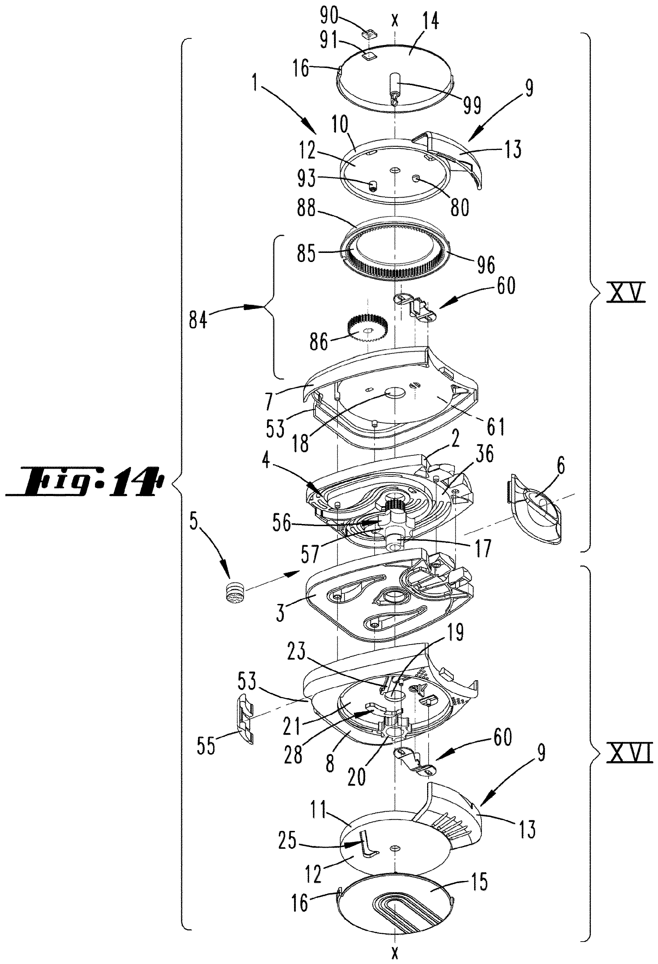

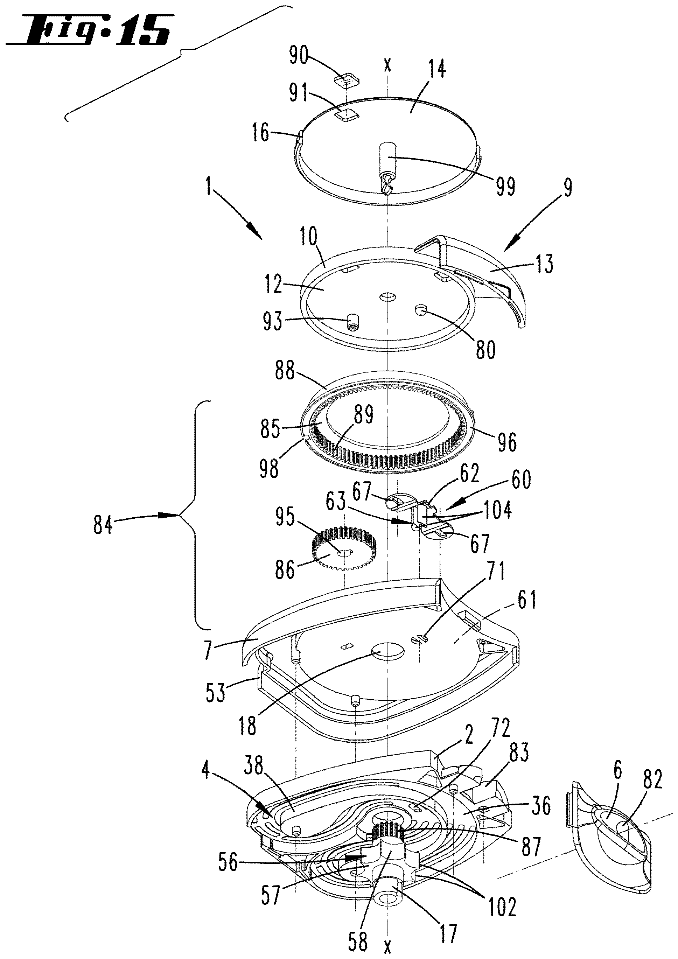

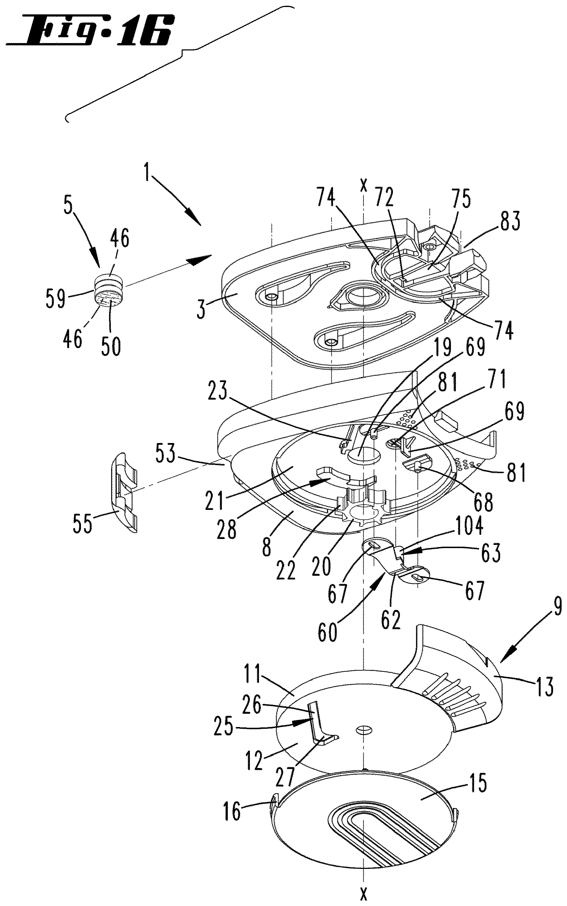

Wie insbesondere aus den explosionsperspektivischen Darstellungen in den Figuren 12 bis 17 ersichtlich, kann die Vorrichtung 1 im Wesentlichen zunächst aus einem Gehäuse-Innenoberteil 2 und einem Gehäuse-Innenunterteil 3 bestehen, die unmittelbar aneinander anliegend zwischen sich die Führungseinrichtung 4 für die Substanzbehälter 5 belassen.

An diesen Gehäuse-Innenteilen 2 und 3 ist ein Mundstück 6 angeordnet, über welches zufolge Einatmen der Inhalationsvorgang durchgeführt werden kann.

Weiter ist Bestandteil der Vorrichtung 1 sowohl ein Gehäuse-Schalenoberteil 7 als auch ein Gehäuse-Schalenunterteil 8, die im Wesentlichen entlang aufeinander zugewandter Randkanten aneinanderliegend zwischen sich die Gehäuse-Innenteile aufnehmen.

Gehäuse-Schalenoberteil 7 und Gehäuse-Schalenunterteil 8 bilden im Wesentlichen zusammen mit dem Mundstück 6 die äußere Kontur der Vorrichtung 1.

Auch ist im Wesentlichen Bestandteil der Vorrichtung 1 eine Verschlusskappe 9 für das Mundstück 6. Die Verschlusskappe 9 ist im Wesentlichen gebildet aus zwei, jeweils dem Gehäuse-Schalenoberteil 7 und dem Gehäuse-Schalenunterteil 8 zugeordneten Verschlusskappenteilen, nämlich einem Verschlusskappen-Oberteil 10 und einem Verschlusskappen-Unterteil 11.

Jedes Verschlusskappenteil weist hierbei einen tellerartigen Deckabschnitt 12 auf und einen von diesem Deckabschnitt 12 nach außen abragenden, in einem Querschnitt L-förmig gebildeten Kappenabschnitt 13.

Entlang der aufeinander zuweisenden freien Randkanten der L-förmigen Kappenabschnitte 13 können diese miteinander verbunden sein, beispielsweise zufolge einer Verklebung oder Verschweißung, so dass sich insgesamt diesbezüglich eine im Querschnitt im Wesentlichen U-förmige Kappe zum Überfangen des Mundstückes 6 ergibt.

Die Deckenabschnitte 12 sind in paralleler Ausrichtung zueinander vorgesehen und relativ zu den Gehäuse-Innenteilen und den Gehäuse-Schalenteilen um eine geometrische Schwenkachse x schwenkverlagerbar.

Das Verschlusskappen-Oberteil 10 kann darüber hinaus von einem tellerartigen Deckelteil 14 überfangen und das Verschlusskappen-Unterteil 11 von einem ebenfalls bevorzugt tellerartigen Bodenteil 15 unterfangen sein.

Die bezüglich des vorbeschriebenen Gehäuses benutzten Begrifflichkeiten "ober" und "unter" beziehungsweise "Boden" oder "Deckel" beziehen sich allein auf die zeichnerischen Darstellungen beispielsweise in den Figuren 12 bis 17. Es ergibt sich bei der vorgeschlagenen Vorrichtung 1 bevorzugt keine bevorzugte Ausrichtung der Vorrichtung zur Erlangung einer korrekten Handhabung. So kann beispielsweise die Oberseite bei der Nutzung durchaus auch die Unterseite der Vorrichtung 1 bilden.

Bodenteil 15 und Deckelteil 14 können, wie auch dargestellt, jeweils einen teilweise um die Schwenkachse x umlaufenden Kragen 16 aufweisen. Deren in Umfangsrichtung weisenden freien Stirnrandkanten können jeweils einen Schwenkanschlag für die Verschlusskappe 9 in der Verschlusskappen-Schließstellung und in der Verschlusskappen-Offenstellung bieten. Entsprechend ist der Schwenkwinkel der Verschlusskappe 9 begrenzt, so beispielsweise auf einen Schwenkwinkel von etwa 50 bis 70 Grad, bevorzugt etwa 60 Grad.

Entlang der geometrischen Schwenkachse x ausgerichtet ist in der Vorrichtung 1 eine Antriebswelle 17 vorgesehen. Diese kann, wie auch bevorzugt relativ zu der Verschlusskappe 9 und dem Gehäuse 52 schwenk- beziehungsweise drehverlagerbar sein. Eine Führung kann die Antriebswelle 17 im Bereich angepasster Bohrungen 18, 19 im Gehäuse-Innenoberteil 2 und Gehäuse-Innenunterteil 3 beziehungsweise in dem Gehäuse-Schalenoberteil 7 und dem Gehäuse-Schalenunterteil 8 erfahren.

Auch kann die Antriebswelle 17 auf einem relativ zu der Antriebswelle 17 feststehenden Achskörper 100 aufgenommen sein. Der Achskörper 100 kann dabei gebildet sein aus jeweils an dem Deckelteil 14 und an dem Bodenteil 15 zentral angeformten und aufeinanderzuweisenden Hohlzapfen 99. Die Hohlzapfen 99 können, wie auch bevorzugt, miteinander verrastet sein, derart, dass das hierdurch insgesamt rastverschlossene Gehäuse 52 bevorzugt nicht mehr zerstörungsfrei öffenbar ist.

Die Antriebswelle 17 ist über die Verschlusskappe 9 ratschenartig antreibbar, derart, dass zufolge einer Schwenkverlagerung der Verschlusskappe 9 insbesondere aus einer das Mundstück 6 verschließenden Grundstellung heraus in eine Offenstellung die Antriebswelle 17 um ein vorgegebenes Winkelmaß drehverlagert wird. Eine Rückschwenkverlagerung der Verschlusskappe 9 aus der Offenstellung in die Schließstellung bewirkt bevorzugt keine Drehmitnahme der Antriebswelle 17.

Hierzu ist die Antriebswelle 17 mit einem Betätigungsrad 20 drehfest verbunden. Dieses sitzt in dem dargestellten Ausführungsbeispiel in einer muldenartigen Vertiefung 21 des Gehäuse-Schalenunterteils 8 zwischen diesem Schalenunterteil und dem Verschlusskappen-Unterteil 11.

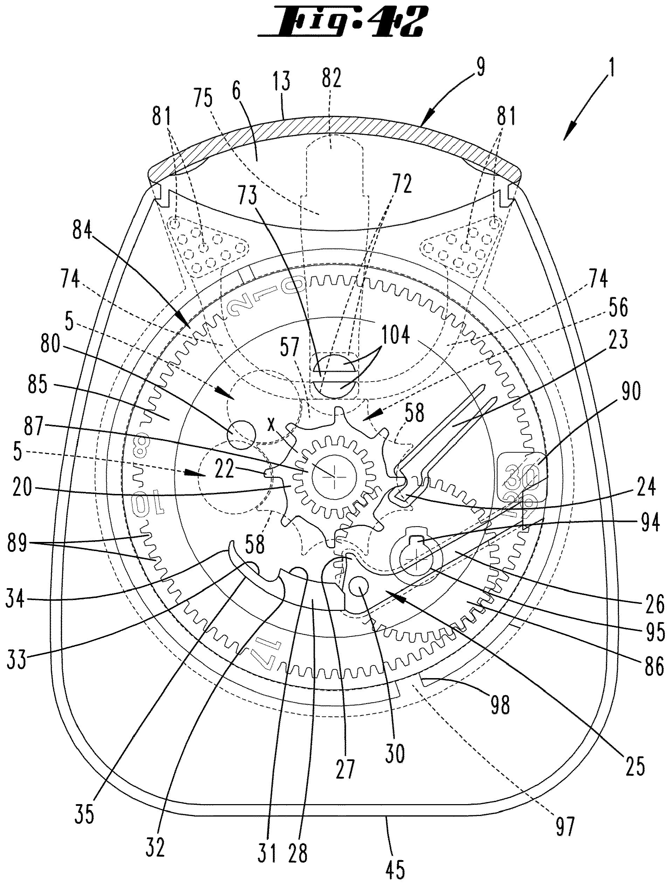

Das Betätigungsrad 20 kann, wie auch dargestellt, über im Wesentlichen radial vorstehende Mitnahmevorsprünge 22 verfügen. In dem dargestellten Ausführungsbeispiel sind über den Umfang gleichmäßig verteilt acht solcher Mitnahmevorsprünge 22 vorgesehen, wobei jeder Mitnahmevorsprung 22 abweichend von einer strengen Radialen zur geometrischen Schwenkachse x einen spitzen Winkel von etwa 20 bis 30 Grad einschließen kann, so dass eine mit Bezug auf eine Draufsicht beispielsweise gemäß Figur 42 gedachte Mittenlinie durch einen Mitnahmevorsprung 22 das Betätigungsrad 20 sekantenartig schneiden kann.

Das Betätigungsrad 20 wirkt im Bereich der Vertiefung 21 mit einer Rücklaufsperre 23 zusammen. Diese kann, wie auch dargestellt, als federnder Abschnitt des Gehäuse-Schalenunterteiles 8, einstückig mit diesem ausgebildet sein.

Die Rücklaufsperre 23 ist mit einer Rastnase 24 versehen, zur Zusammenwirkung mit den Mitnahmevorsprüngen 22 des Betätigungsrades 20, wobei die Rastnase 24 weiter so ausgebildet sein kann, dass diese durch die Mitnahmevorsprünge 22 nur in der vorgegebenen Drehrichtung a des Betätigungsrades 20 überlaufen werden kann. Bei einem solchen Überlaufen federt die Rastnase 24 entsprechend aus.

Entgegen der Drehrichtung a ist ein solches Überlaufen nicht möglich. Vielmehr blockiert die Rastnase 24 der Rücklaufsperre 23 eine Drehung des Betätigungsrades 20 in diese Richtung.

Das Betätigungsrad 20 und über dieses die Antriebswelle 17 ist drehbewegbar zufolge Einwirkung eines Antriebsteiles 25 auf einen der Mitnahmevorsprünge 22 des Betätigungsrades 20. Das Antriebsteil 25 kann, wie auch bevorzugt, Teil der Verschlusskappe 9, insbesondere Teil des Verschlusskappen-Unterteils 11 sein.

Das Antriebsteil 25 kann dabei einstückig und materialeinheitlich mit dem diesbezüglichen Verschlusskappen-Unterteil 11 ausgebildet sein. Hierbei kann weiter das Antriebsteil 25 einen an dem Deckabschnitt 12 wurzelnden Federarm 26 ausweisen, der endseitig eine vorsprungartige Mitnahmenase 27 ausformt. Diese Mitnahmenase 27 ist geeignet zur Zusammenwirkung mit einem Mitnahmevorsprung 22 des Betätigungsrades 20.

Zufolge der Anordnung der Mitnahmenase 27 an einem Federarm 26 ist die Mitnahmenase 27 im Wesentlichen quer zur Längserstreckung des Federarmes 26 federnd ausweichbar gestaltet.

In einer Grundstellung des Antriebsteiles 25, die der Mundstück-Verschlussstellung der Verschlusskappe 9 beispielsweise gemäß Figur 42 entspricht, liegt das Antriebsteil 25 unbelastet und bevorzugt ohne Einfederung in der Vertiefung 21 ein. Erst mit Verschwenken der Verschlusskappe 9 aus der Verschlussstellung in Richtung auf die Offenstellung erfolgt eine gesteuerte Verlagerung des Antriebsteiles 25 entgegen einer sich hierbei einstellenden Rückstellkraft im Bereich des Federarmes 26.

Diese Steuerung ist erreicht über eine relativ zu dem Antriebsteil 25 und dem Betätigungsrad 20 feststehende Kulissenführung 28. Diese kann, wie auch dargestellt, bodenseitig der Vertiefung 21 vorgesehen sein. Die Kulissenführung 28 kann darüber hinaus als eine im Wesentlichen konzentrisch zur Schwenkachse x verlaufende, in Umfangsrichtung gestufte Rippe ausgebildet sein, wobei diese zunächst eine Steuerfläche 29 ausweist, zur Zusammenwirkung mit einem an dem Antriebsteil 25 im Bereich der Mitnahmenase 27 angeformten Steuerzapfen 30.

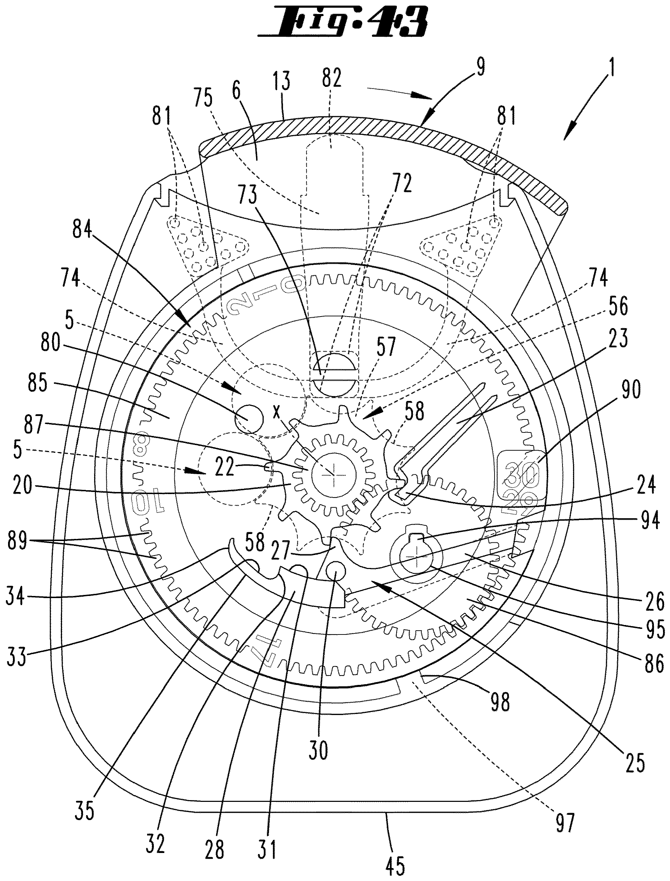

Mit Verschwenken der Verschlusskappe 9 aus der Verschlussstellung gemäß Figur 42 heraus tritt der Steuerzapfen 30 zunächst gegen die Steuerfläche 29, wobei unter weiterer Schwenkverlagerung der Verschlusskappe 9 der Steuerzapfen 30 und hierüber die Mitnahmenase 27 mit Bezug zu der Schwenkachse x nach radial innen gesteuert wird, dies unter Aufbau einer Rückstellkraft im Federarm 26 (vergleiche Figur 43).

Die Mitnahmenase 27 tritt in eine Lücke umfangsmäßig zwischen zwei Mitnahmevorsprünge 22 des Betätigungsrades 20.

Die im Zuge der weiteren Schwenkverlagerung der Verschlusskappe 9 in Richtung auf die Offenstellung entlang einer konzentrisch zur Schwenkachse x verlaufenden ersten Anlagefläche 31 geführte Mitnahmenase 27 schleppt in Anlage an einem Mitnahmevorsprung 22 das Betätigungsrad 20 in Drehrichtung a mit, dies über einen vorbestimmten Winkelbereich, der es ermöglicht, einen Substanzbehälter 5 in eine Entleerungsposition P zu verbringen.

Der über das Antriebsteil 25 bewirkte Drehwinkel des Betätigungsrades 20 ist bevorzugt geringer als der mögliche, anschlagbegrenzte Verschwenkwinkel der Verschlusskappe 9.

Die im Zuge dieser weiteren Schwenkverlagerung der Verschlusskappe 9 über den Federarm 26 mitgeschleppte Mitnahmenase 27 fällt über einen stufenartigen Rücksprung 32 in der Kulissenführung 28 auf eine relativ zu der Schwenkachse x gegenüber der ersten Anlagefläche 31 radial nach außen versetzte zweite Anlagefläche 33. Dieser Abfall der Mitnahmenase 27 ist unterstützt durch die Rückstellkraft des Federarmes 26. Die Mitnahmenase 27 verlässt den Zusammenwirkungsbereich mit dem Mitnahmevorsprung 22 des Betätigungsrades 20, so dass die weitere Schwenkverlagerung der Verschlusskappe 9 keinen weiteren Dreheinfluss auf das Betätigungsrad 20 bewirkt.

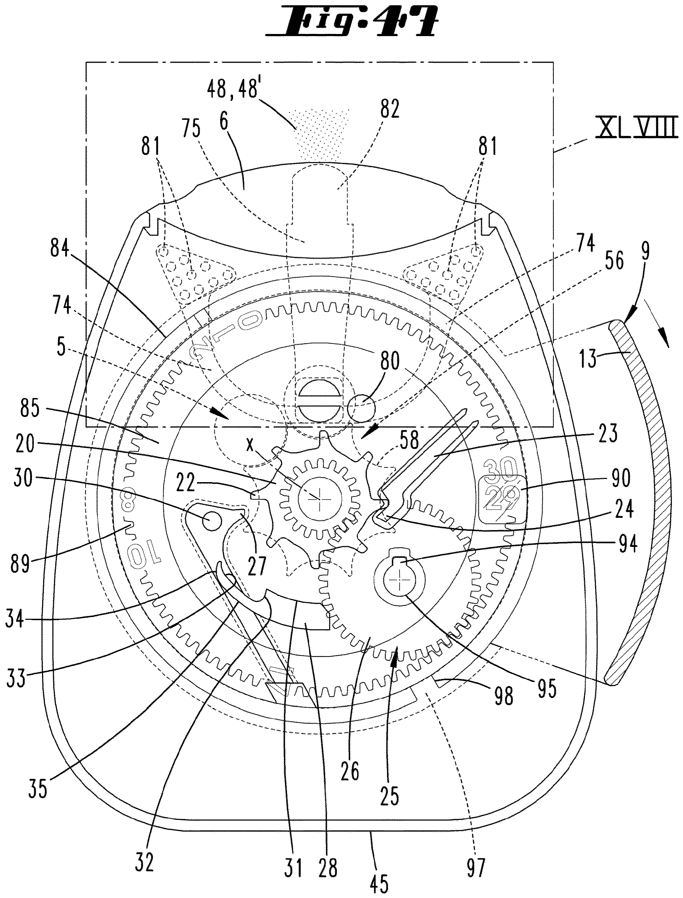

Mit Erreichen der Verschlusskappen-Offenstellung gemäß Figur 47 verlässt der Steuerzapfen 30 die Kulissenführung 28, wonach das Antriebsteil 25 erneut eine bezüglich der Federkräfte entspannte Position einnehmen kann.

Die stufenartige Ausgestaltung der Kulissenführung 28 bewirkt weiter, dass nach einer vollständigen Drehverlagerung des Betätigungsrades 20 um den vorgegebenen Drehwinkel und somit nach Verlagerung eines Substanzbehälters 5 in die Entleerungsposition P zwingend die weitere Verlagerung der Verschlusskappe 9 in Richtung auf die bevorzugt anschlagbegrenzte Offenstellung erfolgen muss. Eine Rückverlagerung der Verschlusskappe 9 kann erst erfolgen, nachdem die Kulissenführung 28 mit Erreichen der Kappen-Offenstellung verlassen wurde (gemäß der Stellung in Figur 47).

Aus dieser Verschlussklappen-Offenstellung heraus wird - bevorzugt nach einem zuvor durchgeführten Inhalationsvorgang - zufolge Verlagerung der Verschlusskappe 9 in Richtung auf die Grundstellung beziehungsweise in Richtung auf die Mundstück-Verschlussstellung gemäß Figur 42 der Steuerzapfen 30 des Antriebsteiles 25 gegen eine endseitige weitere Steuerfläche 34 bewegt, die eine ausfedernde Verlagerung des Steuerzapfens 30 und somit der Mitnahmenase 27 mit Bezug zu der Schwenkachse x nach radial außen bewirkt, so dass der Steuerzapfen 30 hiernach im Zuge der weiteren Schwenkverlagerung der Verschlusskappe 9 in Richtung auf die Mundstück-Verschlussstellung entlang einer gegenüber der ersten und der zweiten Anlagefläche 31, 33 wiederum gegenüber der Schwenkachse x radial nach außen beabstandeten dritten Anlagefläche 35 bewegt wird. Hierbei ergibt sich eine gegenüber der Vorverlagerung des Antriebsteiles 25 entgegengesetzt wirkende Feder-Rückstellkraft im Bereich des Federarmes 26, bis der Steuerzapfen 30 kurz vor Erreichen der Verschlusskappen-Endstellung die Kulissenführung 28 verlässt und die federunbelastete Stellung gemäß Figur 42 wieder einnimmt.

Die zwischen beziehungsweise durch die Gehäuse-Innenober- und Unterteile 2, 3 gebildete Führungseinrichtung 4 bildet einen Vorratsraum 36 für eine Mehrzahl von nicht untereinander verbundenen Substanzbehältern 5. Gehäuse-Innenoberteil 2 und Gehäuse-Innenunterteil 3 bilden die Führungseinrichtung 4 beziehungsweise den Vorratsraum 36 in Erstreckungsrichtung der Schwenkachse x betrachtet jeweils etwa hälftig. Hierbei kann sich je Gehäuse-Innenteil mit Bezug auf einen Querschnitt, in welchem sich die Schwenkachse x als Linie darstellt, ein U-förmiger Abschnitt ergeben, wobei die U-Öffnungen aufeinander zuweisen und die diese U-Öffnung begrenzenden Seitenwandungen 37 an den Stirnflächen aneinander liegen.

Es ergib sich so eine mit Bezug auf die Schwenkachse x ober- und unterseitig wie auch seitlich durch die Seitenwände 37 begrenzte Führungsbahn 38, bei welcher die Seitenwände 37 quer zur Längserstreckung der Führungsbahn 38 bevorzugt angepasst an einen Außendurchmesser d der Substanzbehälter 5 zueinander beabstandet sind.

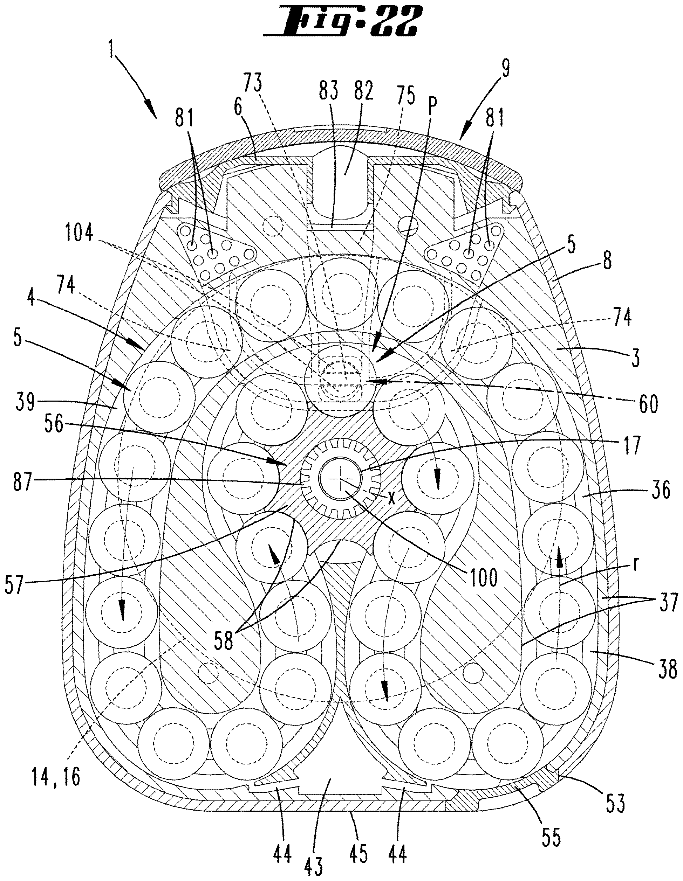

Die Führungsbahn 38 ist, wie beispielsweise aus Figur 22 ersichtlich, mäanderförmig in Art einer Endlosbahn in der Vorrichtung 1 vorgesehen, hierbei im Bereich der Schwenkachse x eine konzentrisch zur Schwenkachse x verlaufende Schleife bildend.

Von dieser Schleife ausgehend erstreckt sich die Führungsbahn 38 beidseitig der Schleife zunächst in Richtung einer dem Mundstück 6 abgewandten Rückseite der Vorrichtung 1, um hiernach sich jeweils über einen nach außen gerichteten Bogen zurück in Richtung des das Mundstück 6 aufweisenden Vorderbereiches der Vorrichtung 1 zu erstrecken. Ein die Schleife umfassender, gegebenenfalls konzentrisch zur Schwenkachse x verlaufender Bogen verbindet die Bahnabschnitte zu einer durchweg gekrümmten Endlosbahn, die bevorzugt keine gerade gestreckt verlaufende Abschnitte aufweist.

Im Bereich des Bahnbodens 39 und / oder der Bahndecke 40 können, wie auch bevorzugt, über die gesamte oder einen Großteil der Führungsbahn 38 Längsnuten 41 vorgesehen sein. So können beispielsweise diesbezüglich zwei oder drei Längsnuten 41 vorgesehen sein, die sich in Längserstreckungsrichtung der Führungsbahn 38 erstrecken und hierbei zueinander quer zur Längserstreckung beabstandet sind.

Auch können diese Längsnuten 41 bereichsweise über Quernuten 42 verbunden sein.

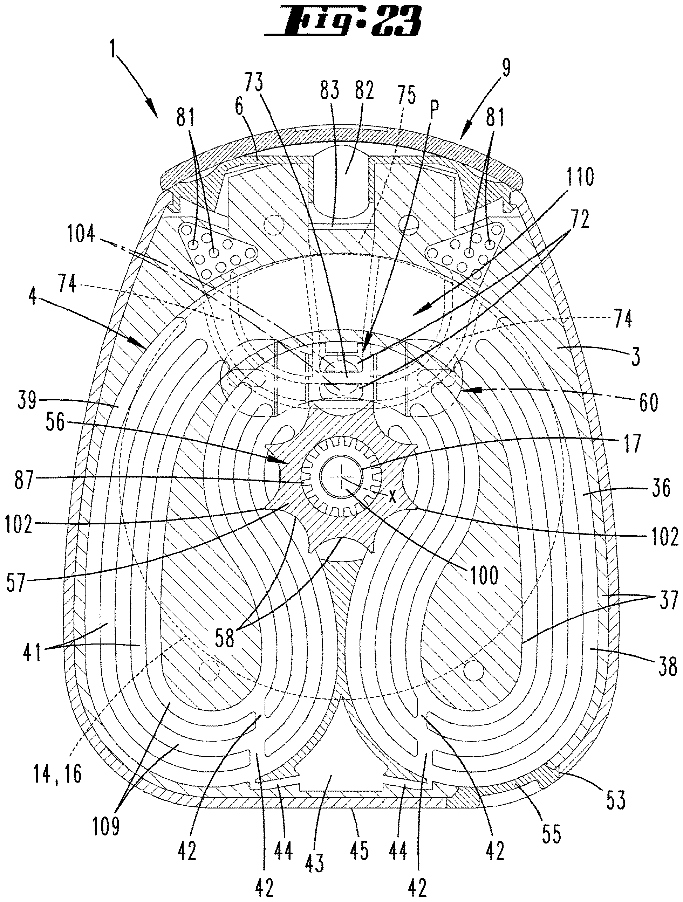

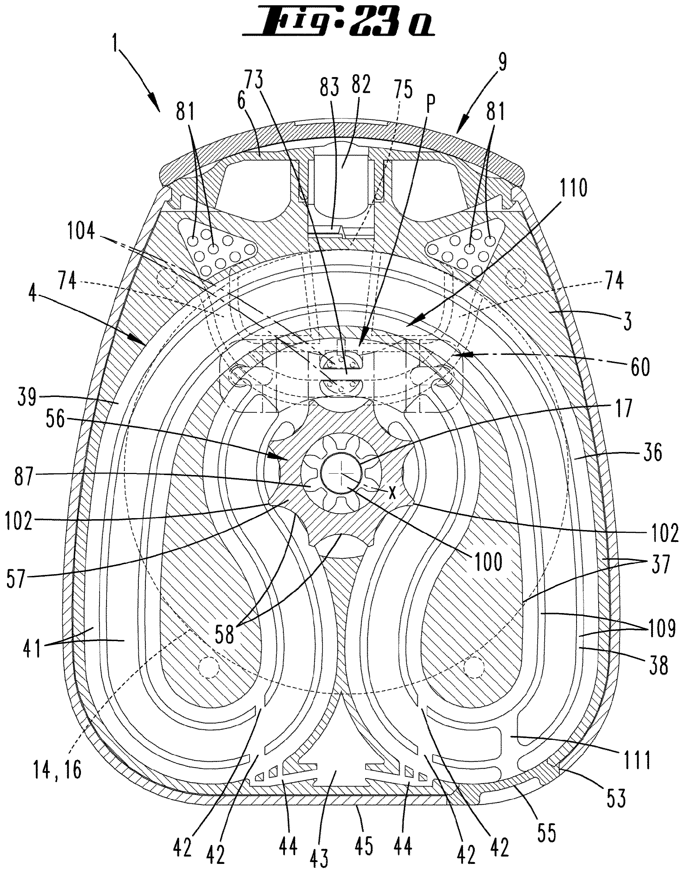

Gemäß beispielsweise der Darstellung in Figur 23 können Längsstege 109, die sich ebenfalls in Längsrichtung erstrecken, die Längsnuten 41 voneinander in der Längsrichtung trennen. In einem dem Mundstück 6 zugeordneten Zenitbereich 110 der Führungseinrichtung 4 können die Längsstege 109 nicht durchlaufend beziehungsweise vorher auslaufend ausgebildet sein. Die Längsnuten 41 können in diesem Bereich entsprechend frei über die gesamte quer zur Längserstreckung der Längsnuten 41 betrachtete Breite in den Bahnboden 39 einlaufen. Es sind in diesem Bereich somit keine zu unterscheidenden Längsnuten gegeben.

Bei dem in Figur 23a gezeigten Ausführungsbeispiel hingegen ist auch der Zenitbereich 110 mit die Längsnuten 41 trennenden Längsstegen 109 durchsetzt.

Auch können gemäß Figur 23a die Längsstege 109 über einen Brückenabschnitt 111, bevorzugt im Bereich einer Einführ- bzw. Gehäuseöffnung 53, untereinander in Querrichtung zur Längserstreckung der Stege verbunden sein.

Über diese Nutstruktur im Boden- und / oder Deckenbereich der Führungsbahn 38 kann in günstiger Weise gegebenenfalls aus einem Substanzbehälter 5 austretende Substanz 48, 48', insbesondere pulverförmige Substanz 48, 48', in die durch die Nuten gebildeten Zwischenräume geführt werden und gegebenenfalls über diese in einen weiter vorgesehenen Sammelraum 43. Dieser Sammelraum 43 kann sich in einem mit Bezug auf das Mundstück 6 rückseitigen Zwickelbereich zwischen den Wendebereichen der Führungsbahn 38 ergeben.

Der Sammelraum 43 ist in diesem Fall bevorzugt über Stichwege 44 mit den zugewandten Abschnitten der Führungsbahn 38 verbunden.

Die diesbezügliche Rückseite der Vorrichtung 1 kann eine Stellfläche 45 für die Vorrichtung 1 anbieten, so dass in diesem Fall der Sammelraum 43 in einen untersten Bereich der Vorrichtung 1 angeordnet ist und die gegebenenfalls sich in den Nuten 41 und 42 ansammelnde Substanz schwerkraftbedingt in Richtung Sammelraum 43 gelangt.

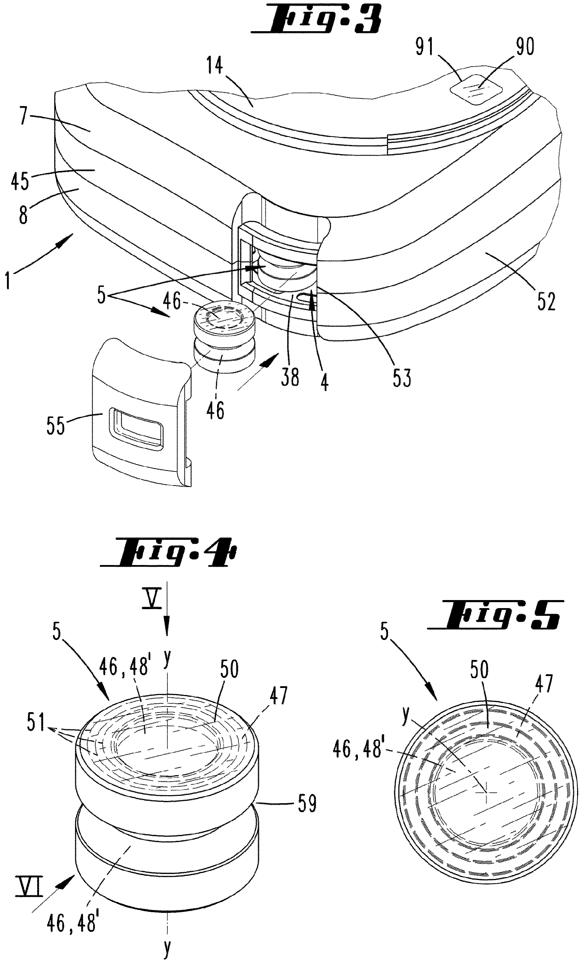

Der insbesondere in den Figuren 4 bis 10 sowie 41 dargestellte Substanzbehälter 5 kann zunächst und im Wesentlichen kreiszylindrisch geformt sein, aufweisend eine Zylinderachse y, die in der Aufnahmestellung des Substanzbehälters 5 in der Vorrichtung 1 beziehungsweise in der Führungseinrichtung 4 gleichgerichtet ausgerichtet ist zu der geometrischen Schwenkachse y beziehungsweise der Drehachse der Antriebswelle 17.

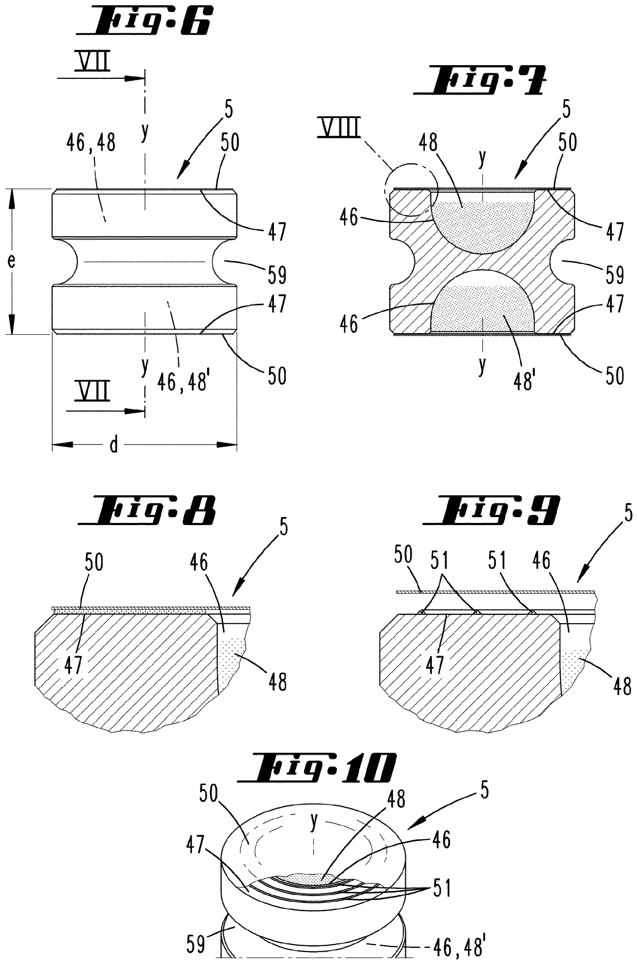

Der Außendurchmesser d kann, wie dargestellt, größer gewählt sein als die in Achsrichtung betrachtete Höhenerstreckung des Substanzbehälters 5. So kann der Durchmesser d etwa dem 1,2- bis 1,5-Fachen der axialen Höhe e entsprechen (vergleiche Figur 6).

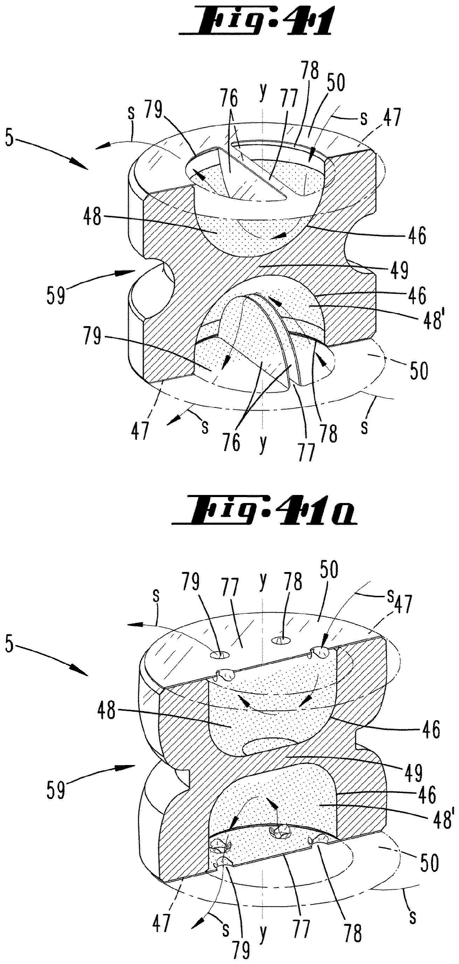

Auch der Substanzbehälter 5 ist bevorzugt aus einem Hartkunststoff gefertigt, beispielsweise Polypropylen oder Polyethylen. Dieser weist zwei in Erstreckungsrichtung der Zylinderachse y gegenüberliegende und konzentrisch zur Zylinderachse y ausgerichtete Teilbereiche 46 beziehungsweise Kavitäten auf. Diese können, wie beispielsweise in Figur 41 dargestellt, im Wesentlichen etwa als halbkugelförmige Vertiefungen gebildet sein. Die jeweilige Öffnung ergibt sich in der quer zur Achse y ausgerichteten jeweiligen Stirnfläche. Jede Kavität kann ausgelegt sein, zur Aufnahme einer Substanzmenge von beispielsweise 2 bis 250 µg, weiter beispielsweise 10 bis 100 µg.

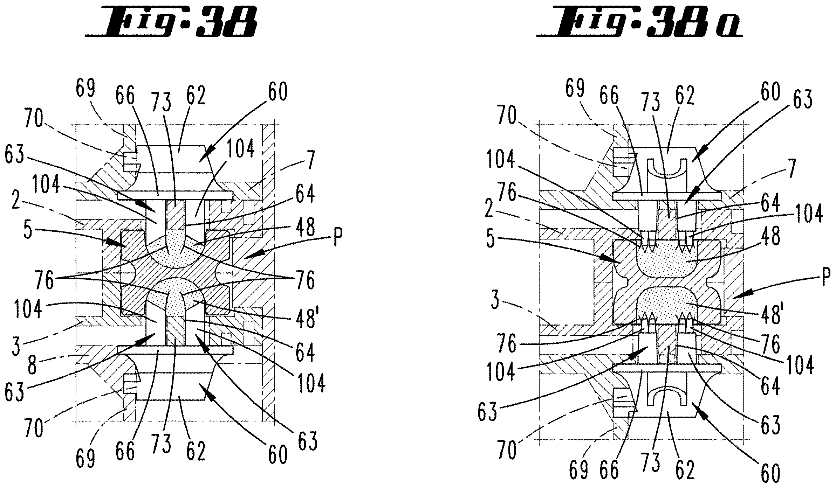

Gemäß den Darstellungen in den Figuren 38a und 41a kann ein Teilbereich 46 des Substanzbehälters 5 auch als im Wesentlichen topfartige Vertiefung ausgebildet sein, mit einer im Wesentlichen relativ zu der Zylinderachse y zylindrischen Topfwandung und einem quer zur Achse y verlaufenden Topfboden. Der Übergang von Topfwandung in den Topfboden ist verrundet. Auch durch diese Querschnittsgeometrie gemäß Figur 41a ist ein günstiges Ausräumen der Kavität erreichbar. Der Topfboden weist einen sich quer zu der Achse y ebenflächig erstreckenden Bereich auf oder ist zumindest mit einem im Vergleich zu dem Übergang von der Topfwandung in den Topfboden sehr viel größeren Radius gekrümmt verlaufend gebildet.

Die Teilbereiche 46 sind ausgebildet zur Aufnahme jeweils einer Substanz 48, 48'.

Durch die Trennung der Teilbereiche 46 zueinander unter Einziehung eines bevorzugt mit Bezug zu der Höhe e mittig eingezogenen Bodens 49, können in den Teilbereichen 46 unterschiedliche Substanzen 48, 48' aufgenommen sein.

Die Kavitäten beziehungsweise Teilbereiche 46 sind jeweils mit einer öffenbaren beziehungsweise durchstechbaren Abdeckung 50 überdeckt. Diese Abdeckungen 50 versiegeln den jeweiligen Teilbereich 46 und die hierin aufgenommene Substanz 48, 48'.

Bei der Abdeckung 50 kann es sich, wie auch bevorzugt, um eine Folie handeln, beispielsweise eine Aluminiumfolie. Diese Folie ist bevorzugt mit dem Stirnrand 47 des Substanzbehälters 5 verschweißt.

Hierzu kann darüber hinaus der Stirnrand 47 umlaufend in Achsrichtung vorstehende Rippen 51 aufweisen, die nach Auflage der folienartigen Abdeckung 50 im Zuge des Schweißvorganges, insbesondere Ultraschweißvorganges, schmelzen und unter Vergleichmäßigung in der Oberfläche die Anhaftung der Abdeckung 50 erbringen.

In der vorrichtungsfesten Führungseinrichtung 4 ist eine Mehrzahl solcher hinsichtlich des Aufbaus und der Abmessungen bevorzugt gleicher Substanzbehälter 5 aufgenommen. Die jeweils aufgenommene Substanz in den Substanzbehältern 5 kann jedoch beispielsweise bezüglich der Zusammensetzung und/oder Menge und/oder Dosierung unterschiedlich sein.

Gemäß dem dargestellten Ausführungsbeispiel können dreißig solcher Substanzbehälter 5 in der Endlos-Führungsbahn 38 aufgenommen sein, wobei die Substanzbehälter 5 in Längserstreckung der Führungsbahn 38 betrachtet im Wesentlichen unmittelbar aneinander zur Anlage kommen. Dabei sind die Substanzbehälter 5 jeweils seitlich durch die Seitenwände 37 der Führungseinrichtung 4 geführt.

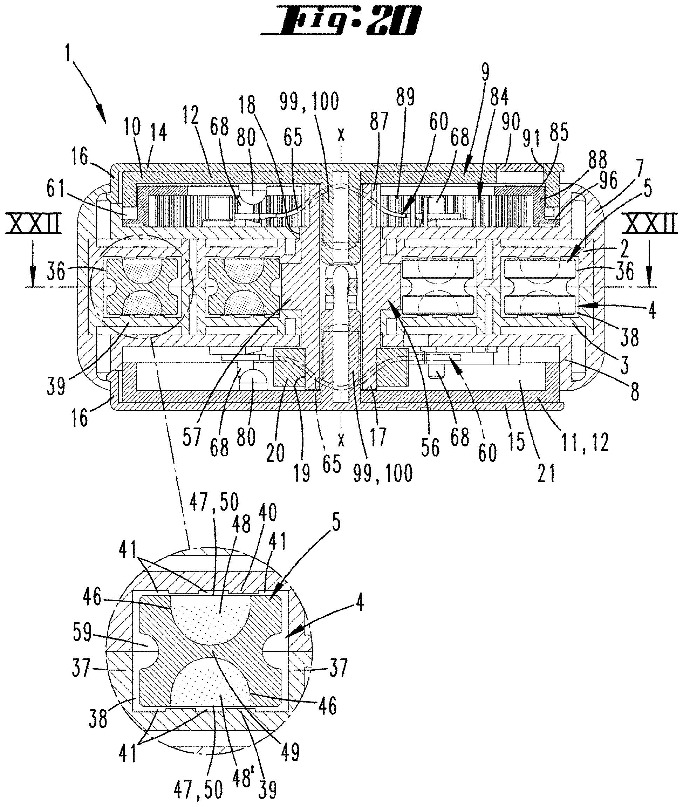

Über ihre Stirnränder 47 erfahren die Substanzbehälter 5 jeweils eine Abstützung an den gegenüber den Längs- und Quernuten 41 und 42 erhabenen Auflageflächen von Bahnboden 39 und Bahndecke 40 (vergleiche Figur 20, insbesondere die dazu gehörige Lupendarstellung).

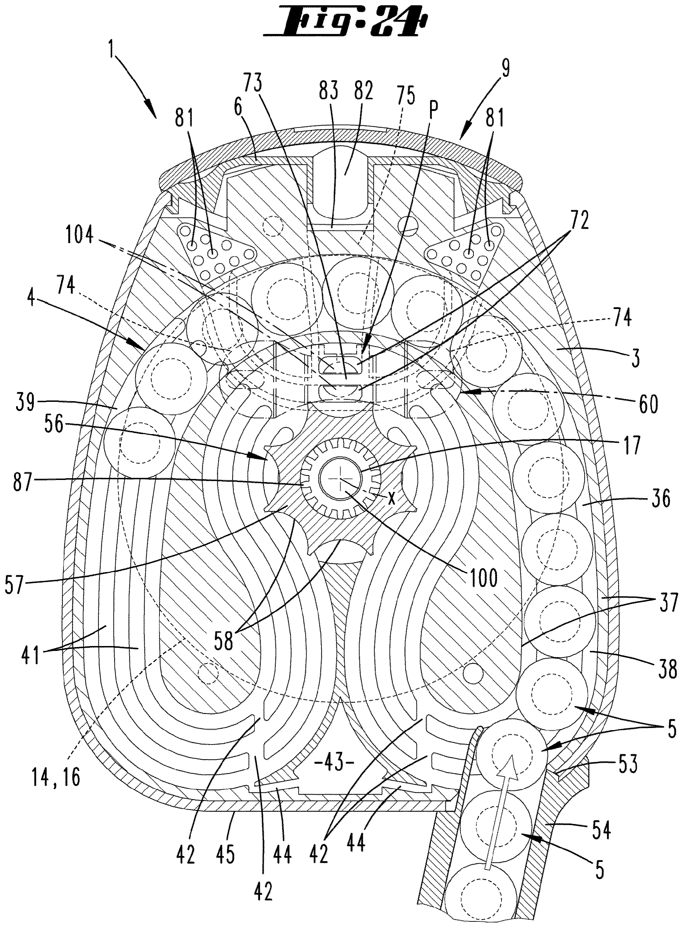

Wie aus der Darstellung in Figur 24 ersichtlich, können die Substanzbehälter 5 auch erst nach im Wesentlichen vollständigem und funktionsfähigem Zusammenbau der Vorrichtung 1 in die Führungseinrichtung 4 beziehungsweise in die Führungsbahn 38 eingeführt werden. Hierzu ist an dem Gehäuse 52 der Vorrichtung 1, etwa zugeordnet der Stellfläche 45 und somit weiter bevorzugt zugeordnet einem rückwärtigen Wendeabschnitt der Führungsbahn 38, eine Einführ- beziehungsweise Gehäuseöffnung 53 vorgesehen.

An diese Öffnung 53 ist eine Einführhilfe, beispielsweise in Form einer Einführschiene 54, ansetzbar, über welche die in Hintereinanderanordnung in der Schiene aufgenommenen Substanzbehälter 5 allein durch sich unter den Substanzbehältern 5 fortpflanzenden Druck in die Führungsbahn 38 verbracht werden können. Bevorzugt entspricht die Anzahl der in der Einführschiene 54 zunächst aufgenommen Substanzbehältern 5 der in der Führungsbahn 38 beziehungsweise der Führungseinrichtung 4 maximal aufnehmbaren Substanzbehältern 5.

Mit dem letzten eingeführten Substanzbehälter 5 wird eine untereinander nicht verbundene Endloskette aus Substanzbehältern 5 in der Führungseinrichtung 4 geschlossen. Der zuletzt eingeführte Substanzbehälter 5 wirkt in der Endloskette in Art eines Schlusssteines.

Die Einlaufrichtung der Substanzbehälter 5 im Zuge der Bestückung der Vorrichtung 1 unter Nutzung beispielsweise einer solchen Einführschiene 54 entspricht der Verlagerungsrichtung r der Substanzbehälter 5 innerhalb der Vorrichtung 1 bei üblicher Nutzung der Vorrichtung 1.

Die Einführ- beziehungsweise Gehäuseöffnung 53 ist abschließend, das heißt nach vollständiger Bestückung der Vorrichtung 1 mit der vorgegebenen Anzahl an Substanzbehältern 5, durch ein Verschlussteil 55 verschlossen. Das Verschlussteil 55 kann mit dem umlaufenden Gehäuserand beispielsweise verklebt oder verschweißt sein. Gegebenenfalls ist diesbezüglich auch eine Rastverbindung möglich. Wesentlich ist hierbei, dass das Verschlussteil 55 nach einem entsprechenden Verschluss nicht mehr zerstörungsfrei entfernbar ist.

Das Verschlussteil 55 bildet in der Verschlussstellung wandungsinnenseitig einen Teil der Führungsbahn 38 beziehungsweise der Seitenwand 37.

Die Substanzbehälter 5 werden in der Führungseinrichtung 4 beziehungsweise der Führungsbahn 38 über ein Antriebselement 56 in Längserstreckungsrichtung der Führungsbahn 38 bewegt, derart, dass ein entleerter Substanzbehälter 5 aus der Entleerungsposition P heraus verlagert wird und ein unmittelbar nachfolgender, Substanz 48 und 48' in seinen Teilbereichen 46 bevorratender Substanzbehälter 5 in diese Entleerungsposition P nachrückt.

Die Entleerungsposition P ist in dem dargestellten Ausführungsbeispiel erreicht im Zenit der die Schwenkachse x umgreifenden Schleife der Führungsbahn 38.

Das Antriebselement 56 kann, wie auch dargestellt, ein sternradartiges Antriebsrad 57 sein, das drehfest auf der Antriebswelle 17 über das Betätigungsrad 20 antreibbar ist. Bevorzugt ist in der Vorrichtung 1 nur ein solches Antriebselement 56 beziehungsweise Antriebsrad 57 vorgesehen.

Das Antriebsrad 57 ist mit radial offenen Aufnahmeausformungen 58 versehen, die über den Umfang betrachtet durch radial vorstehende Antriebszähne 102 beidseitig begrenzt und durch die Antriebszähne 102 zueinander distanziert sind. Über dem Umfang gleichmäßig verteilt können, wie im Ausführungsbeispiel dargestellt, acht solcher Aufnahmeausformungen 58 vorgesehen sein. Diese sind bevorzugt gleichgestaltet in Form konkaver Randausnehmungen, insbesondere kreisabschnittförmiger Randausnehmungen, deren Radius bevorzugt angepasst ist an den Außendurchmesser d eines Substanzbehälters 5.

Mit den Aufnahmeausformungen 58 erfasst das Antriebselement 56 die Substanzbehälter 5 im Bereich der Führungsbahn-Schleife. Hierbei werden beispielsweise sieben solcher Substanzbehälter 5 erfasst beziehungsweise durch das Antriebsrad 57 geführt und in der Führungsbahn 38 zufolge Drehverlagerung des Antriebsrades 57 geleitet. Durch den sich in der Endloskette unter den Substanzbehältern 5 fortpflanzenden Anlagedruck werden dabei alle Substanzbehälter 5 bei einsprechender Drehung des Antriebsrades 57 weiter bewegt.

Der Drehwinkel des Antriebsrades 57 zum Wechseln der Substanzbehälter 5 in der Entleerungsposition P ist unter anderem abhängig von der Anzahl an Aufnahmeausformungen 58. Bei acht Aufnahmeausformungen 58 ergibt sich ein entsprechender Drehwinkel von bevorzugt etwa 45 Grad.

Wie erwähnt, ist der Schwenkwinkel der Verschlusskappe 9, über welche Schwenkverlagerung über die Antriebswelle 17 auch auf das Antriebsrad 57 Einfluss genommen wird, größer gewählt als der erlaubte Drehwinkel des Antriebsrades 57. Durch die vorbeschriebene Kulissenführung 28 gelangt das Antriebsteil 25 nach Durchführung einer 45 Grad Drehung des Antriebsrades 57 außer Eingriff zu dem Betätigungsrad 20.

Es ist so sichergestellt, dass mit jeder Öffnungsbewegung der Verschlusskappe 9 eine Verlagerung der Substanzbehälter-Kette nur um einen Behälter in Verlagerungsrichtung r vorgenommen wird.

Wie weiter insbesondere aus den Darstellungen in den Figuren 6, 7 und 41 zu erkennen, kann der Substanzbehälter 5 bezogen auf die Zylinderachse y etwa mittig eine außen umlaufende, quer zu der Zylinderachse y ausgerichtete Nut 59 aufweisen. Diese Nut 59 kann mit Bezug auf einen Querschnitt, in welchem sich die Zylinderachse y als Linie darstellt, eine halbkreisförmige sich nach außen öffnende Kontur aufweisen (vergleiche Figur 7 oder 41).

In alternativer Ausgestaltung kann der nach radial innen gerichtete Nutgrund der Nut 59 als ein kreiszylindrischer Wandungsabschnitt gebildet sein, von welchem ausgehend sich die Nutwandungen in einem Querschnitt gemäß Figur 41a nach radial außen jeweils entlang einer gekrümmten Linie erstrecken, derart, dass sich eine trichterartige Erweiterung der Nut 59 bis in die Behälterwandung ergibt.

Die radiale Tiefe der Nut 59 beziehungsweise ein diesbezüglicher Radius der umlaufenden halbkreisförmigen Vertiefung kann bevorzugt so gewählt sein, dass sich insgesamt annähernd eine Vergleichmäßigung der Wandungsstärke des Substanzbehälters 5 ergibt, weiter insbesondere bezüglich der konzentrisch zur Zylinderachse y umlaufenden Wandung.

Durch die gegebene Taillierung des Substanzbehälters 5 ist sogenannten Einfallerscheinigungen, wie diese bei zu hohen Wandungsstärken bei Hartkunststoffprodukten auftreten können, entgegengewirkt. Darüber hinaus ergibt sich hierdurch auch eine Material- und hierüber eine Gewichtsersparnis.

Des Weiteren kann die Nut 59 auch genutzt sein, zur Führung der Substanzbehälter 5 in der Vorrichtung 1, insbesondere in der Führungseinrichtung 4, wozu eine oder beide Seitenwände 37 der Führungsbahn 38 mittig ihrer in Erstreckungsrichtung der Schwenkachse x betrachteten Höhe eine in Richtung auf die gegenüberliegende Seitenwand weisende Rippe oder dergleichen aufweisen kann, die in die Nut 59 des Substanzbehälters 5 führend eingreift. So kann darüber die Führung der Substanzbehälter 5 in der Führungseinrichtung 4 gegebenenfalls allein über diese in die Nuten 59 eingreifenden Rippen erfolgen. Die Stirnränder 47 der Substanzbehälter 5 können dabei zu dem Bahnboden 39 und/oder zu der Bahndecke 40 beabstandet sein.

Zugeordnet der Entleerungsposition P eines Substanzbehälters 5 ist eine Einstecheinrichtung 60 vorgesehen, zur kontrolliert gezielten Öffnung der substanzbehälterseitigen Abdeckung 50.

Entsprechend der Ausbildung von zwei, jeweils Substanzen 48, 48' aufweisenden Teilbereichen 46 in dem Substanzbehälter 5 sind bevorzugt auch zwei Einstecheinrichtungen 60 vorgesehen. Diese liegen in Erstreckungsrichtung der Schwenkachse x in Gegenüberlage.

Dabei kann eine Einrichtung 60 in der Vertiefung 21 des Gehäuse-Schalenunterteiles 8 angeordnet sein und die weitere Einstecheinrichtung 60 in einer solchen Vertiefung 61 im Gehäuse-Schalenoberteil 7.

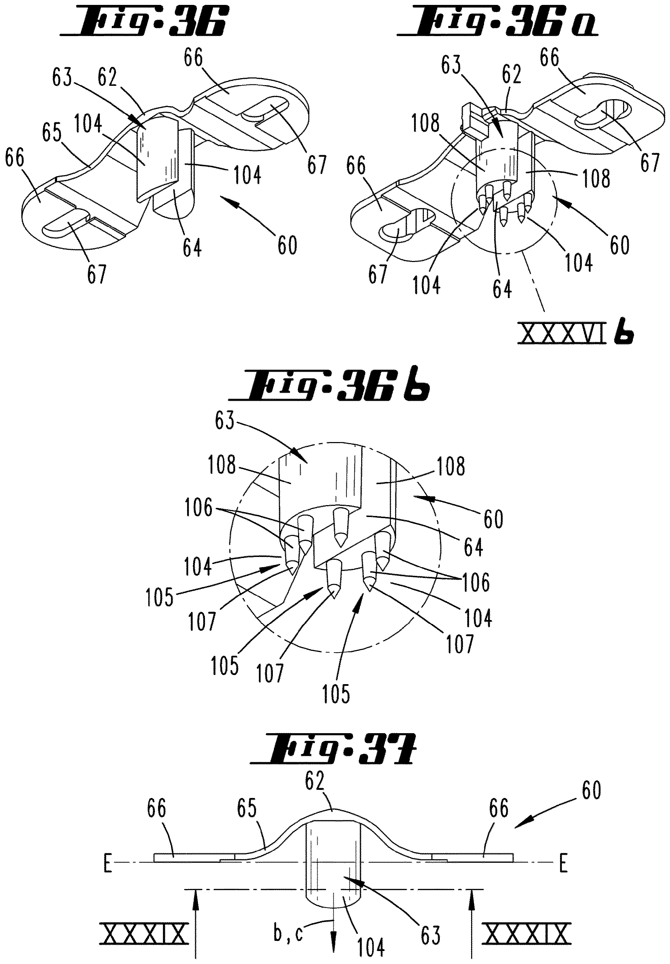

Eine Einstecheinrichtung 60 ist beispielhaft in den Figuren 36 bis 39 dargestellt.

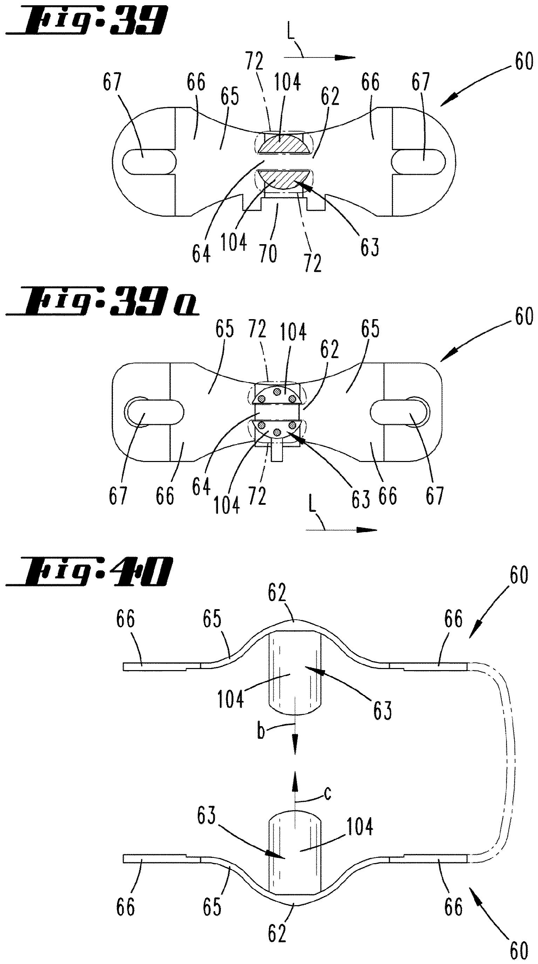

Jede Einstecheinrichtung 60 weist zunächst ein Halteteil 62 auf, an welchem ein Einstechmittel 63 befestigt ist. Bevorzugt sind zugeordnet einem Einstechmittel 63 zwei gesonderte Einstechbereiche 104 vorgesehen, die mit Bezug auf einen Querschnitt gemäß der Darstellung in Figur 39 beispielsweise kreissegmentartig gestaltet sein können.

Wie beispielsweise aus den Figuren 36a und 36b ersichtlich kann jeder Einstechbereich 104 alternativ zwei oder mehr - hier beispielsweise drei - Einstechspitzen 105 aufweisen. Diese können stirnseitig an kreisegmentartigen Sockeln 108 angeordnet sein. Die Einstechspitzen 104 ragen bevorzugt frei über eine Stirnfläche des Sockels 108 hinaus.

Mit Bezug auf eine Längserstreckung L einer Einstecheinrichtung 60 insgesamt sind die Einstechbereiche 104 quer zu dieser Längserstreckung L zueinander beabstandet, wobei bei einer kreissegmentartigen Ausbildung der Einstechbereiche 104 die Flachseiten der Kreissegmente aufeinander zuweisen. Es ergibt sich so entsprechend zwischen den Einstechbereichen 104 ein schlitzartiger Freiraum 64, der sich bevorzugt über die gesamte Erstreckungslänge der Einstechbereiche 104 senkrecht zur Längserstreckung L bis zum Halteteil 62 erstreckt.

Die von dem Halteteil 62 abweisenden freien Endbereiche der Einstechbereiche 104 können klingenartig gespitzt ausgebildet sein, mit einer Klingenspitze bevorzugt jeweils im Zenitbereich der Kreissegmente.

Bei Ausbildung von Einstechspitzen 105 ist eine dornartige Ausbildung bevorzugt, mit einem bevorzugt zylindrischen Bereich 106, über welchen die Einstechspitze 105 an dem Sockel 108 angebunden ist, und einem anschließenden Spitzenbereich 107. Der Spitzenbereich 107 kann ausgehend von dem zylindrischen Bereich 106 konisch sich zum freien Ende hin verjüngend ausgebildet sein.

Jede Einstecheinrichtung 60 weist bevorzugt ein gesondertes Halteteil 62 auf. Wie beispielhaft in Figur 40 anhand der stichpunktierten Linie dargestellt, können aber auch beide Einstecheinrichtungen 60 ein gemeinsames Halteteil 62 aufweisen.

Das Halteteil 62 einer Einstecheinrichtung 60 kann darüber hinaus, wie auch bevorzugt, kombiniert mit einer Kunststofffeder 65 ausgebildet sein. Diese Kunststofffeder 65 weist zwei in Längserstreckung L entgegen gerichtete Federarme 66 auf. Dabei erstrecken sich die Federarme 66 mit Bezug auf die Längserstreckung L in dem jeweiligen Endbereich bevorzugt annähernd innerhalb einer gemeinsamen, quer zur Erstreckungsrichtung der Einstechmittel 63 ausgerichteten Auflageebene E. Der in Längserstreckung L betrachtete mittige Verbindungsbereich der Federarme 66 erstreckt sich in einer Seitenansicht gemäß Figur 37, in der sich eine Einstechrichtung b beziehungsweise c linienartig darstellt und beide Federarme 66 sich in ihrer Längserstreckung abbilden, konkav gewölbt, wobei das Halteteil 62 bevorzugt mittig der Längserstreckung L zwischen den Federarmen 66 angeordnet ist. Die Einstechmittel 63 sind bevorzugt unterseitig der durch die konkave Gestaltung im Bereich des Halteteils 62 gegebenen Gewölbedecke angeordnet und durchsetzen bevorzugt die vorbeschriebene gemeinsame Auflageebene E der beiden Federarme 66 (vergleiche Figur 37).

Die Federarme 66 können ausgehend von ihren freien Enden, jeweils in den, die Auflageebene E anbietenden Bereichen, langlochartige Führungsausnehmungen 67 aufweisen, die jeweils mit einem gehäusefesten Zapfen 68 zusammenwirken können. Über diese Zapfen 68 kann eine Festlegung der Einstecheinrichtung 60 an dem jeweiligen Gehäuseteil (Gehäuse-Schalenoberteil 7 oder Gehäuse-Schalenunterteil 8) erreicht sein. Darüber hinaus kann hierüber auch zugleich eine Führung des Halteteils 62 bei einem Einstechvorgang gegeben sein.

Eine solche Führung kann auch gegeben sein durch Zusammenwirkung zwischen einem gehäuseseitigen Führungsfortsatz 69 und einer im Bereich des Halteteils 62 vorgesehenen randseitigen Führungsausklinkung 70.

Jedenfalls ist jeweils in Einstechrichtung b beziehungsweise c der jeweiligen Einstecheinrichtung 60 eine exakte Führung insbesondere der Einstechbereiche 104 gegeben, so dass die aufeinander zugerichteten Einstechrichtung b, c über den gesamten Verlagerungsweg bevorzugt senkrecht gerichtet ist zur Auflageebene E.

Jede Einstecheinrichtung 60 wird bei entsprechender Beaufschlagung gegen die Rückstellkraft der Kunststofffeder 65 in Richtung der Schwenkachse x durch die jeweilige Abdeckung 50 des Substanzbehälters 5 zum Öffnen der Teilbereiche 46 gedrückt. Hierzu sind im jeweiligen Vertiefungsboden des die Einstecheinrichtung 60 tragenden Gehäuse-Schalenoberteils 7 und des Gehäuse-Schalenunterteils 8 Führungsdurchbrüche 71 vorgesehen, durch welche die Einstechbereiche 104 tauchen können.

Auch in dem Gehäuse-Innenoberteil 2 und dem Gehäuse-Innenunterteil 3 sind entsprechende Durchbrüche 72 ausgebildet. Diese sind zunächst angepasst an den Außendurchmesser der Einstechmittel 63 bohrungsartig vorgesehen, hierbei mittig entlang einer Durchmesserlinie einen die Bohrung in zwei Teilabschnitten trennenden Steg 73 aufweisend. Der Steg 73 ist hinsichtlich seiner quer zum Durchmessermaß betrachteten Breite angepasst an das entsprechende Abstandsmaß der Einstechbereiche 104 im Bereich der schlitzartigen Führung 64 zueinander. Ein solcher Steg kann auch im Bereich der Führungsdurchbrüche 71 in den Gehäuseschalenteilen vorgesehen sein.

Der Steg 73 bietet zunächst insbesondere im Zuge des Einstechvorganges eine Stabilisierung und Führung der Einstechbereiche 104. Darüber hinaus bietet der Steg 73 zugleich eine Trennung zwischen Ansaugkanälen 74 und einem Austragskanal 75.

Jeder Kavität beziehungsweise jedem Teilbereich 46 des in der Entleerungsposition P befindlichen Substanzbehälters 5 sind über die jeweiligen Stege 73 getrennt Ansaugkanäle 74 und ein Auftragskanal 75 zugeordnet.

Zufolge der vorbeschriebenen Anordnung und Ausgestaltung der Einstecheinrichtung 60 wirken die Einstechmittel 63 beziehungsweise die Einstechbereiche 104 bevorzugt in entgegengesetzten Einstechrichtungen b und c, jeweils gerichtet entlang der Schwenkachse x. Bevorzugt wirken die Einstechmittel 63 bei entsprechender Belastung zum Durchbruch der Abdeckungen 50 in aufeinander zuweisenden Richtungen.

Durch die Einstechbereiche 104 können sich in jeder Abdeckung 50 des in der Entleerungsposition P befindlichen Substanzbehälters 5 bei einer Ausbildung der Einstechbereiche 104 beispielsweise gemäß der Darstellung in Figur 36 oder 39 zwei kreissegmentartige Stanzabschnitte 76 ergeben, die bevorzugt mittig entlang einer Durchmesserlinie des Substanzbehälters 5 beziehungsweise der Abdeckung 50 über einen verbleibenden Abdeckungssteg 77 nach innen in den jeweiligen Teilbereich 46 einlappen (vergleiche insbesondere Figur 41).

Wie anhand der Figuren 38a und 41a dargestellt, bewirken die Einstechspitzen 105 der Einstecheinrichtung 60 gemäß beispielsweise Figur 36a eine lochartige Stanzung der behälterseitgen Abdeckung 50. Die sich entsprechend ergebenden Stanzlöcher (Öffnungen 78 und 79) können jeweils ein Durchmessermaß aufweisen, welches kleiner gewählt sein kann als beispielsweise 2mm, weiter bevorzugt kleiner als 1,5mm, so gegebenenfalls bis hin zu 0,5mm oder weniger. Es ergeben sich bevorzugt stecknadelgroße Öffnungen 78 und 79.

Zwischen zwei Gruppen an Stanzlöchern, welche Gruppen sich zum einen aus Öffnungen 78 und zum anderen aus Öffnungen 79 zusammensetzen, ergibt sich auch hier ein nicht durchsetzter bzw. nicht gestanzter, mittiger Abdeckungssteg 77.

Es kann sich dabei weiter zugeordnet jedem Teilbereich 46 eine Öffnung 78 zum Eintritt der Luftströmung s aus den Ansaugkanälen 74 und eine Öffnung 79 zum Austritt der mit Substanz 48 beziehungsweise 48' aus dem Teilbereich 46 versetzten Luftströmung s in den Austragskanal 75 ergeben.

Bevorzugt liegt der die Ansaugkanäle 74 zu dem Austragskanal 75 trennende Steg im Durchbruch 72 dichtend auf der Abdeckung 50 beziehungsweise dem sich nach Öffnung der Abdeckung 50 ergebenden Abdeckungssteg 77 auf, so dass eine Zwangsführung der Luftströmung s durch die Öffnung 78 und durch den Teilbereich 46 gegeben ist.

Die Ausformung des Teilbereiches 46 als gegebenenfalls halbkugelartige Vertiefung unterstützt den Räumungseffekt über die Luftströmung s. Es ergeben sich keine strömungsmäßigen Todbereiche. Durch die in Richtung auf den Teilbereich-grund einlappenden Stanzabschnitte 76 wird die Luftströmung s bodennah durch den Teilbereich 46 geführt, was das vollständige Räumen des Teilbereiches 46 unterstützt.

Bevorzugt werden beide Einstecheinrichtungen 60 gleichzeitig belastet und entlastet. Auch kann diesbezüglich eine wahlweise Belastung der einen oder beider Einstecheinrichtungen 60 vorgenommen werden.

Dargestellt in den zeichnerischen Darstellungen ist eine Ausführungsform, bei welcher die Bewegung beider Einstecheinrichtungen 60 synchron erfolgt zufolge Schwenkverlagerung der Verschlusskappe 9.

Hierzu kann unterseitig des Verschlusskappen-Oberteils 10 und des Verschlusskappen-Unterteils 11, jeweils gerichtet in Richtung auf die jeweils zugewandte Vertiefung 21 beziehungsweise 61, ein Nocken 80 angeformt sein, der im Zuge der Schwenkbewegung der Verschlusskappen 9, bevorzugt nachgeordnet einer Vorverlagerung des Substanzbehälters 5 in die Entleerungsposition P, die Einstechmittel 63 gegen die Rückstellkraft der Kunststofffeder 65 in Einstechrichtung b und c niederdrücken. Hierbei beaufschlagen die Nocken 80 die Einstecheinrichtung 60 bevorzugt im Bereich des jeweiligen Halteteils 62.

Der vorbeschriebene Vorlauf des Substanzbehälters 5 zur Erlangung der Entleerungsposition P ist beispielsweise bereits mit einer Schwenkverlagerung der Verschlusskappe 9 um etwa 45 Grad erreicht. Unter Aufhebung der Mitschleppbewegung zwischen Verschlusskappe 9 und Betätigungsrad 20 kann die Verschlusskappe 9 hiernach frei weiter in Richtung auf die vollständige Öffnungsstellung verschwenken, wobei im Zuge dieser Schwenkbewegung die Nocken 80 die Halteteile 62 der Einstecheinrichtungen 60, diese beaufschlagend, überlaufen. Zufolge des Vorlaufes des Substanzbehälters 5 ist hierbei sichergestellt, dass ein neuer, nicht geräumter Substanzbehälter 5 in der vorbeschriebenen Entleerungsposition P vorliegt. Erst hiernach erfolgt eine gezielte Durchstoßung der Abdeckungen 50.

Zum Ende der Verschwenkbewegung der Verschlusskappe 9 verlassen die Nocken 80 den Einflussbereich auf die Einstecheinrichtungen 60, welche sich zufolge der Rückstellkraft der Kunststofffedern 65 wieder zurück in ihrer Ausgangsstellung bewegen. Hierbei fahren die Einstechmittel 63 beziehungsweise Einstechbereiche 104 wieder aus den Teilbereichen 46 des Substanzbehälters 5 heraus, zur entsprechenden Freigabe der Öffnungen 78 und 79 beziehungsweise zur strömungsmäßigen Verbindung dieser Öffnungen mit den Ansaug- und Austragskanälen 74, 75.

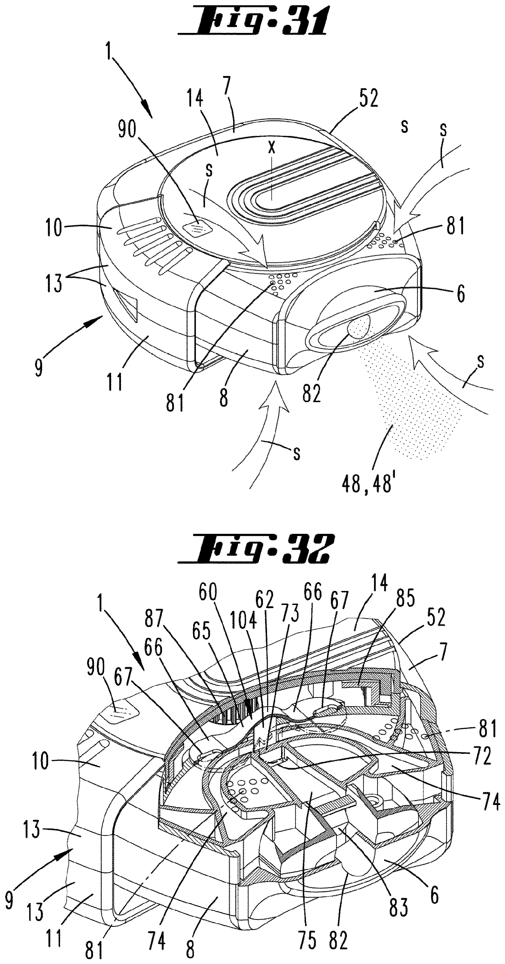

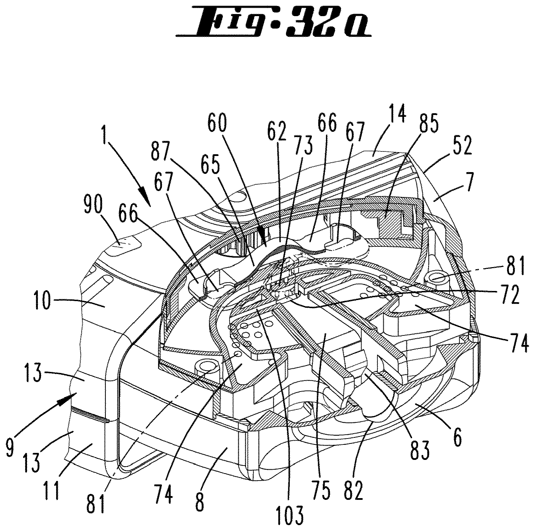

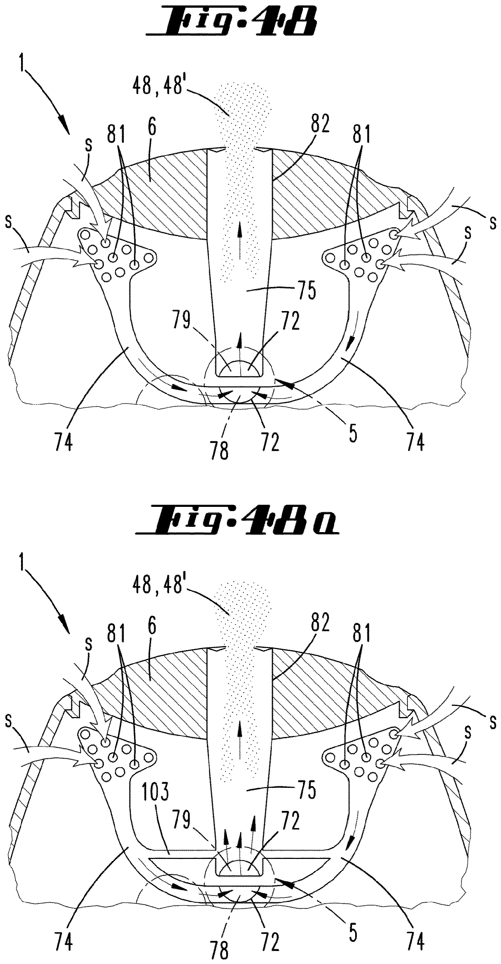

Jeden Teilbereich 46 des in der Entleerungsposition befindlichen Substanzbehälters 5 sind zwei Ansaugkanäle 74 zugeordnet. Deren Ansaugöffnungen 81 sind beidseitig des Mundstückes 6 in dem jeweiligen Gehäuse-Schalenoberteil 7 beziehungsweise dem Gehäuse-Schalenunterteil 8 ausgebildet, während die Ansaugkanäle 74 im Wesentlichen sich in dem Gehäuse-Innenoberteil 2 beziehungsweise dem Gehäuse-Innenunterteil 3 ausformend erstrecken können (vergleiche beispielsweise Figuren 32 und 48).

Es ergeben sich so insgesamt in der Vorrichtung 1 vier Ansaugkanäle 74 mit vier Ansaugöffnungen 81, welche Ansaugöffnungen 81 so beidseitig neben dem Mundstück 6 positioniert sind, dass diese erst nach einem Aufschwenken der Verschlusskappe 9 in Richtung auf die Offenstellung freigelegt werden. In der verschlossenen Grundstellung der Vorrichtung 1 liegen die Ansaugöffnungen 81 geschützt unter der Verschlusskappe 9 versteckt.

Die beiden Ansaugkanäle 74 einer Kavität treffen sich bevorzugt unmittelbar im Bereich des durch den Steg 73 getrennten Durchbruches 72 (vergleiche auch Figur 48).

In alternativer Ausgestaltung kann, insbesondere und bevorzugt im Zusammenhang mit der Nutzung einer Einstecheinrichtung 60 mit Einstechspitzen 105 gemäß den Figuren 36a, 36b und 39a, ein die Ansaugkanäle 74 in Luftströmungsrichtung s vor dem Substanzbehälter 5 bzw. vor der Entleerungsposition P verbindender Bypass 103 vorgesehen sein (siehe Fig. 32a und 48a). Durch diesen Querkanal (Bypass 103) kann ein Teil der angesaugten Luft im Zuge eines Inhalationsvorganges unmittelbar und ohne Durchsetzung der Kavitäten des Substanzbehälters 5 in den Austragskanal 75 strömen. Ein solcher Bypass 103 kann, wie auch bevorzugt, jedem Paar der Ansaugkanäle 74 zugeordnet sein.

Weiter ist jeder Kavität beziehungsweise jedem Teilbereich 46 eines in der Entleerungsstellung befindlichen Substanzbehälters 5 ein Austragskanal 75 zugeordnet. Entsprechend ergeben sich in der Vorrichtung 1 insgesamt zwei Austragskanäle 75, die zunächst ausgehend von der zugeordneten Durchbruchöffnung getrennt zueinander in Richtung auf das Mundstück 6 geführt sind, dies insbesondere in eine Richtung etwa senkrecht zu der vorbeschriebenen rückwärtigen Stellfläche 45.

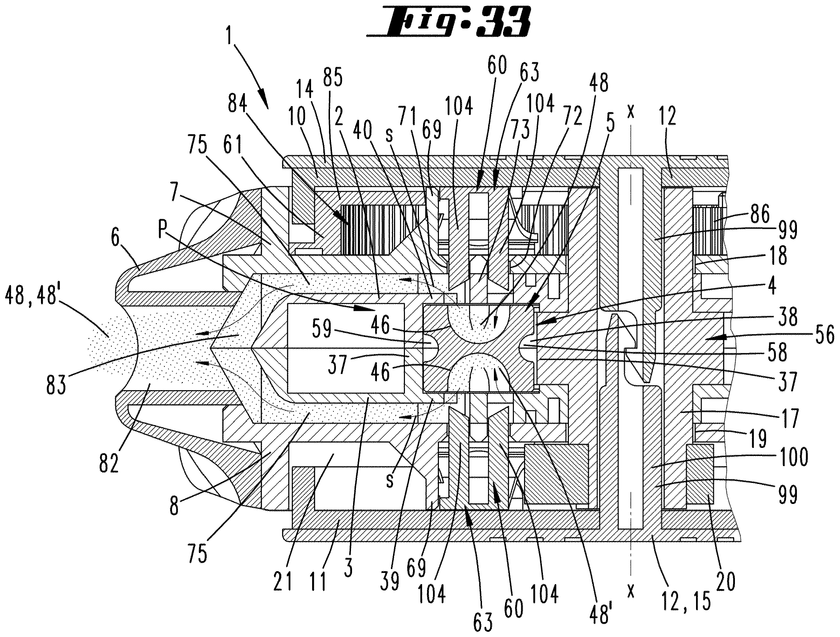

Die beiden Austragskanäle 75 sind unmittelbar im Übergang zu dem Mundstückkanal 82 zusammengeführt (vergleiche beispielsweise Figur 33). In diesem Zusammenführungsbereich 83 kann gegebenenfalls ein Verwirbelungselement oder dergleichen vorgesehen sein.

Entsprechend der vorbeschriebenen Ausgestaltung und Trennung der Austragskanäle 75 werden im Zuge eines Inhalationsvorganges nach Öffnen der Abdeckungen 50 durch die Einstecheinrichtungen 60 und Aufbau einer Luftströmung s zufolge Ansaugen beziehungsweise Einatmen über das Mundstück 6 die Substanzen 48 und 48' getrennt aus den jeweiligen Teilbereichen 46 geräumt und erst unmittelbar vor Übergang in den Atembereich des Benutzers, insbesondere im Zusammenführungsbereich 83 im Wurzelbereich des Mundstückes 6, zusammengeführt und vermengt beziehungsweise verwirbelt.

Die Vorrichtung 1 ist weiter ausgelegt und ausgebildet zur Zählung der durchgeführten oder noch verbleibenden Entleerungsvorgänge beziehungsweise Inhalationsvorgänge. Hierzu ist ein Zählwerk 84 vorgesehen.

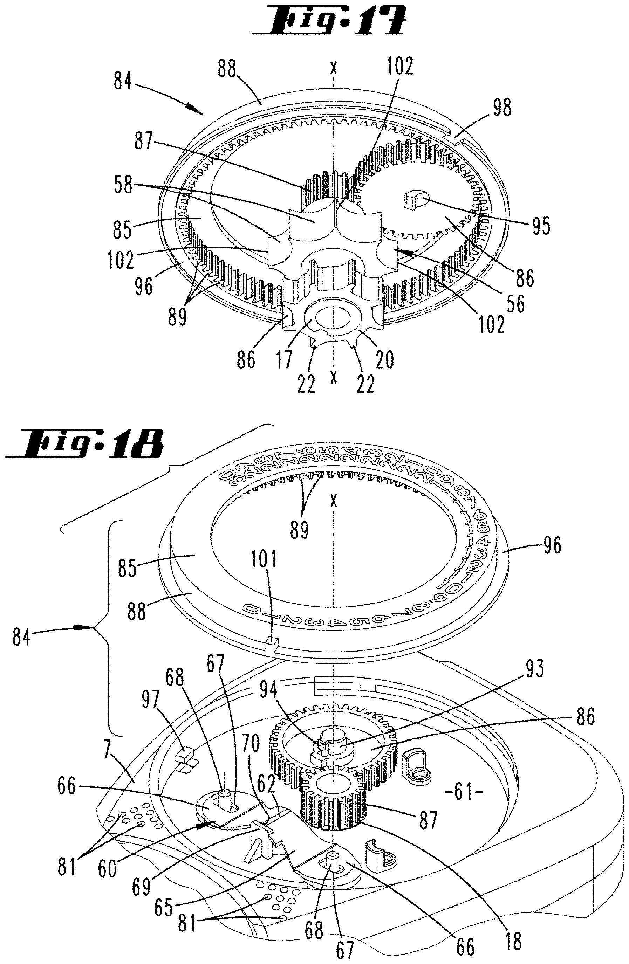

Das Zählwerk 84 weist im Wesentlichen, wie beispielsweise aus der Detaildarstellung in Figur 17 zu erkennen, ein ringförmiges Zählrad 85, ein Übertragungszahnrad 86 und ein Antriebsritzel 87 auf. Das Antriebsritzel 87 ist drehfest auf der Antriebswelle 17 angeordnet und kämmt mit einer Außenverzahnung des Übertragungszahnrades 86. Die Außenverzahnung des Übertragungszahnrades 86 wiederum kämmt mit einer innenseitig eines umlaufenden Kragens 88 des Zählrades 85 ausgebildeten Innenverzahnung 89.

Das so gebildete, getriebeartige Zählwerk 84 ist im Wesentlichen angeordnet zwischen dem Gehäuse-Schalenoberteil 7 und dem zugeordneten Verschlusskappen-Oberteil 10.

Im Bereich einer oberseitigen, ringförmigen Fläche des Zählrades 85 sind Symbole, insbesondere Ziffern aufgebracht. Bevorzugt entspricht die Anzahl der Ziffern der Anzahl der in der Führungseinrichtung 4 der Vorrichtung 1 aufnehmbaren Substanzbehälter 5. So kann, gemäß dem dargestellten Ausführungsbeispiel, eine Ziffernfolge von 0 bis 30 vorgesehen sein.

Die aktuelle Drehausrichtung des Zählrades 85 und entsprechend die darzustellende aktuelle Zahl an beispielsweise noch vorhandenen unverbrauchten Substanzbehältern 5 oder alternativ an bereits verbrauchten Substanzbehältern 5 ist über ein transparentes Fenster 90 im Deckelteil 16 von außen für den Benutzer sichtbar. Das Fenster 90 verschließt einen angepassten Durchbruch 91 im Deckelteil 16. Auch das Verschlusskappen-Oberteil 10 weist einen solchen Durchbruch 92 auf, der in der Mundstück-Verschlussstellung der Verschlusskappe 9 in Bezug zu der Schwenkachse x in fluchtender Übereinanderlage zu dem Durchbruch 91 und dem Fenster 90 im Deckelteil 14 sich befindet. Zudem kann ein weiterer, in Umfangsrichtung versetzt ausgebildeter Durchbruch 92' vorgesehen sein, durch welchen die Anzeige auch in der Verschlusskappenoffenstellung erkennbar ist.

Im Zuge der Verschlusskappen-Schwenkbewegung sind die Ziffern des Zählrades 85 nicht über das Fenster 90 sichtbar, da sich der ansonsten geschlossene Deckabschnitt 12 des Verschlusskappen-Oberteils 10 zwischen Zählrad 85 und Fenster 90 stellt. Mit vollendeter Rückschwenkbewegung der Verschlusskappe 9 in die Verschlussstellung des Mundstückes 6 ist die gegenüber vor Einleitung einer Verschwenkung der Verschlusskappe 9 aus der Verschlussstellung in die Öffnungsstellung sichtbare Zahl um 1 erhöht oder alternativ um 1 verringert.

Die Verschlusskappen-Schwenkbewegung bewirkt demzufolge sowohl eine Verlagerung der Substanzbehälter 5 innerhalb der Führungseinrichtung 4 um eine Position, um so den nächsten Substanzbehälter 5 in die Entleerungsposition P zu verbringen, als auch das Öffnen der Abdeckungen 50 beider Teilbereiche 46 des in der Entleerungsposition P befindlichen Substanzbehälters 5 und darüber hinaus eine Änderung der Anzeige des Zählwerke 84.

Das Übertragungszahnrad 86 des Zählwerks 84 ist geführt auf einem Achszapfen 93 des Gehäuse-Schalenoberteils 7. Dabei erstreckt sich eine geometrische Drehachse des Übertragungszahnrades 86 gleichgerichtet zur Schwenkachse x.

Der Achszapfen 93 weist endseitig, entsprechend beabstandet zu dem Vertiefungsboden, in welchem der Achszapfen 93 wurzelt, einen Radialvorsprung 94 auf. Das Übertragungszahnrad 86 weist eine entsprechend angepasste, schlüssellochartige zentrale Durchbrechung auf, die es erlaubt, dass Übertragungszahnrad 86 nur in einer Drehausrichtung auf den Achszapfen 93 aufzuschieben. In der Betriebsstellung unterläuft die Nabe des Übertragungszahnrades 86 den Radialvorsprung 94 des Achszapfens 93, so dass das Übertragungszahnrad 86 frei drehbar ist.

Das Zählrad 85 weist entlang seines umlaufenden Kragens 88 einen nach außen weisenden Radialkragen 86 auf. Dieser Radialkragen 96 ist in der Betriebsstellung überfangen von einem im Bereich einer die Vertiefung 61 umfassenden Gehäusewandung nach radial innen abragenden, eine weitere Ausrichtungsausformung 97 ausbildenden Steg. Im Bereich des Radialkragens 96 des Zählrades 85 ist eine an die Ausrichtungsausformung 97 angepasste randoffene Ausnehmung 98 vorgesehen.

Zufolge der vorbeschriebenen Ausgestaltungen sind sowohl das Zählrad 85 als auch das Übertragungszahnrad 86 nur in einer vorgegebenen Winkelausrichtung relativ zueinander und / oder relativ zu dem Antriebsritzel 87 montierbar.

Dabei kann die Einbauposition insbesondere des Zählrades 85 derart sein, dass erst nach Einführen der Substanzbehälter 5 in die ansonsten betriebsbereite Vorrichtung 1 das Zählrad 85 so ausgerichtet wird, dass durch das Fenster 90 beispielsweise die dann noch maximale Anzahl durchführbarer Inhalation beziehungsweise unbenutzter Substanzbehälter 5 erkennbar ist, so gemäß dem gezeigten Ausführungsbeispiel die Ziffer 30. Durch die in die Führungseinrichtung 4 beispielsweise über die Einführschiene 54 sukzessive eingeführten Substanzbehälter 5 wird das in einer vorgegebenen Drehposition eingesetzte Zählrad 85 über die vorbeschriebene Getriebeanordnung mitgeschleppt und in die exakte Ausgangsposition versetzt.

Nach einem Öffnen der Kavitäten des in der Endlosreihe letzten Substanzbehälters 5 - und bevorzugt einer hiernach durchgeführten Inhalation - kann die Vorrichtung 1 zur Vorbereitung einer vermeintlich nächsten Inhalation gesperrt sein.

So kann in dieser Position das Zählwerk 84, wie auch bevorzugt, Null anzeigen. Das entsprechend in diese Position gedrehte Zählrad 85 kann dabei mit einer im Bereich des Radialkragens 96 angeformten Anschlagrippe 101 gegen einen gehäusefesten Abschnitt, beispielsweise gegen die Ausrichtungsausformung 97, sperrend treten.

Hierdurch kann eine Blockierung des getriebeartigen Zählwerks 84 und hierüber der Antriebswelle 17 erreicht sein, so dass bei einem Versuch eines Aufschwenkens der Verschlusskappe 9 aus der Verschlussstellung heraus in Richtung auf die Offenstellung das Antriebsteil 25 gegen die blockierte Antriebswelle 17 beziehungsweise das drehfest damit verbundene Betätigungsrad 20 tritt.

Die Vorrichtung 1 ist nach Entleerung aller Substanzbehälter 5 gesperrt und bevorzugt nicht weiter nutzbar. Durch die bevorzugte Endlos-Aneinanderreihung der Substanzbehälter 5 in der Führungseinrichtung 4 würde ohne eine solche Blockierung nach dem letzten Substanzbehälter 5 der erste, bereits entleerte Substanzbehälter erneut in die Entleerungsposition P verbracht werden. Einer solchen Fehlbedienung ist durch die vorbeschriebene Blockierung entgegengewirkt.

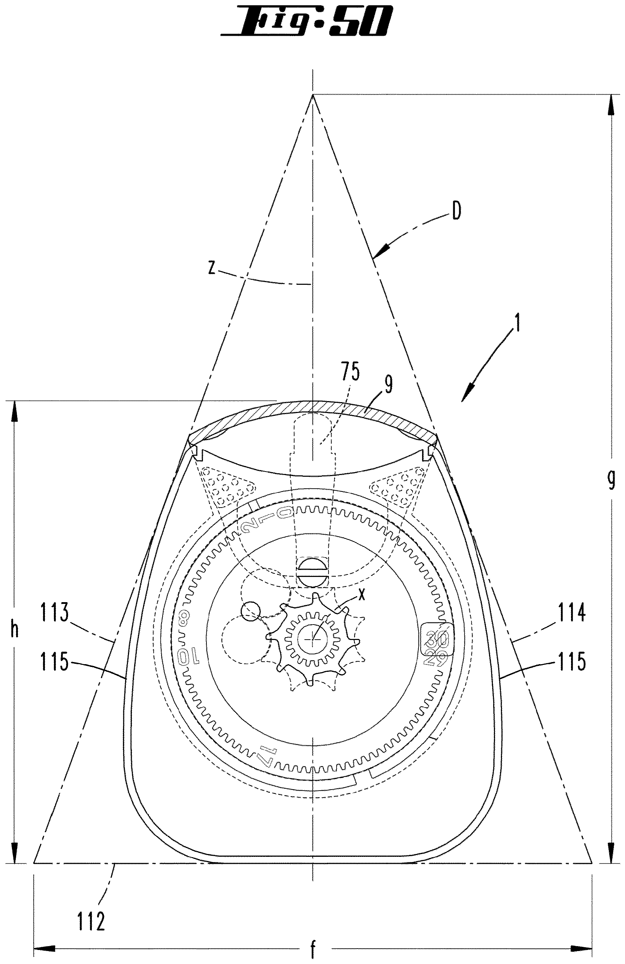

Wie weiter beispielsweise auch aus der schematischen Darstellung in Figur 50 zu erkennen, kann die äußere Grundrissform der Vorrichtung, in welchem Grundriss sich die Schwenkachse x als Punkt darstellt, im Wesentlichen aus einem spitzwinkligen, gleichschenkligen Dreieck D resultieren, aufweisend eine Basislinie 112 mit einer Basisbreite f und eine Symmetrieachse z, die die Schwenkachse x der Vorrichtung 1 aufnimmt und den Austragskanal 75 in dessen Längsausrichtung mittig durchsetzt.

Die Höhe g des Dreiecks D entlang der Symmetrieachse z kann etwa dem 1,2- bis 3-Fachen oder mehr, weiter bspw. etwa dem 1,5- bis 2-Fachen der Basisbreite f entsprechen.

Die Basislinie 112 des Dreiecks D tangiert dabei bevorzugt die durch die Stellfläche 45 gegebene Grundrisslinie der Vorrichtung 1, während die Schenkel 113 und 114 des Dreiecks D die den Stellflächenbereich mit dem Mundstückbereich verbindenden Seitenwandabschnitte 115 des Gehäuse-Schalenoberteils 7 und des Gehäuse-Schalenunterteils 8 tangential berühren.

Die Höhe h der Vorrichtung 1, ausgehend von der Stellfläche 45 und innerhalb des Dreiecks D liegend entlang der Symmetrieachse z bis zur Verschlusskappe 9 betrachtet, kann etwa dem 0,7- bis 1,3-Fachen oder mehr, weiter beispielsweise dem 0,8- bis 1-Fachen der Basisbreite f des Dreiecks D entsprechen.

**Liste der Bezugszeichen**

| | | | |
|---|---|---|---|
| 1 | Vorrichtung | 43 | Sammelraum |
| 2 | Gehäuse-Innenoberteil | 44 | Stichweg |
| 3 | Gehäuse-Innenunterteil | 45 | Stellfläche |
| 4 | Führungseinrichtung | 46 | Teilbereich |
| 5 | Substanzbehälter | 47 | Stirnwand |
| 6 | Mundstück | 48 | Substanz |
| 7 | Gehäuse-Schalenoberteil | 48' | Substanz |
| 8 | Gehäuse-Schalenunterteil | 49 | Boden |
| 9 | Verschlusskappe | 50 | Abdeckung |
| 10 | Verschlusskappen-Oberteil | 51 | Rippe |
| 11 | Verschlusskappen-Unterteil | 52 | Gehäuse |
| 12 | Deckabschnitt | 53 | Einführ- bzw. Gehäuseöffnung |
| 13 | Kappenabschnitt | 54 | Einführschiene |
| 14 | Deckelteil | 55 | Verschlussteil |
| 15 | Bodenteil | 56 | Antriebselement |
| 16 | Kragen | 57 | Antriebsrad |
| 17 | Antriebswelle | 58 | Aufnahmeausformung |
| 18 | Bohrung | 59 | Nut |
| 19 | Bohrung | 60 | Einstecheinrichtung |
| 20 | Betätigungsrad | 61 | Vertiefung |
| 21 | Vertiefung | 62 | Halteteil |
| 22 | Mitnahmevorsprung | 63 | Einstechmittel |
| 23 | Rücklaufsperre | 64 | schlitzartiger Freiraum |
| 24 | Rastnase | 65 | Kunststofffeder |
| 25 | Antriebsteil | 66 | Federarm |
| 26 | Federarm | 67 | Führungsausnehmung |
| 27 | Mitnahmenase | 68 | Zapfen |
| 28 | Kulissenführung | 69 | Führungsfortsatz |
| 29 | Steuerfläche | 70 | Führungsausklinkung |
| 30 | Steuerzapfen | 71 | Führungsdurchbruch |
| 31 | erste Anlagefläche | 72 | Durchbruch |
| 32 | Rücksprung | 73 | Steg |
| 33 | zweite Anlagefläche | 74 | Ansaugkanal |
| 34 | Steuerfläche | 75 | Austragskanal |
| 35 | dritte Anlagefläche | 76 | Stanzabschnitt |
| 36 | Vorratsraum | 77 | Abdeckungssteg |
| 37 | Seitenwand | 78 | Öffnung |
| 38 | Führungsbahn | 79 | Öffnung |
| 39 | Bahnboden | 80 | Nocken |
| 40 | Bahndecke | 81 | Absaugöffnung |
| 41 | Längsnut | 82 | Mundstückkanal |
| 42 | Quernut | 83 | Zusammenführungsbereich |
| 84 | Zählwerk | | |
| 85 | Zählrad | | |
| 86 | Übertragungszahnrad | | |
| 87 | Antriebsritzel | a | Drehrichtung |
| 88 | Kragen | b | Einstechrichtung |
| 89 | Innenverzahnung | c | Einstechrichtung |
| 90 | Fenster | d | Außendurchmesser |
| 91 | Durchbruch | e | Höhe |
| 92 | Durchbruch | f | Basisbreite |
| 92' | Durchbruch | g | Höhe |
| 93 | Achszapfen | h | Höhe |
| 94 | Ausrichtungsausformung | r | Transportrichtung |
| 95 | Durchbrechung | s | Luftströmung |
| 96 | Radialkragen | x | Schwenkachse |
| 97 | Ausrichtungsausformung | y | Zylinderachse |
| 98 | Ausnehmung | z | Symmetrieachse |
| 99 | Hohlzapfen | | |
| 100 | Achskörper | D | Dreieck |
| 101 | Anschlagrippe | E | Auflageebene |
| 102 | Antriebszahn | L | Längserstreckung |
| 103 | Bypass | P | Entleerungsposition |
| 104 | Einstechbereich | | |
| 105 | Einstechspitze | | |
| 106 | zylindischer Bereich | | |
| 107 | Spitzenbereich | | |
| 108 | Sockel | | |
| 109 | Längssteg | | |
| 110 | Zenitbereich | | |
| 111 | Brückenabschnitt | | |
| 112 | Basislinie | | |
| 113 | Schenkel | | |
| 114 | Schenkel | | |
| 115 | Seitenwandabschnitt | | |

## Patentansprüche

1. Vorrichtung (1) zum Inhalieren pulverförmiger Substanzen (48), wobei die Vorrichtung (1) ein Mundstück (6), eine Einstecheinrichtung (60) mit einem ein Einstechmittel (63) aufweisenden Halteteil (62) zur Öffnung eines Substanz (48, 48') enthaltenen Teilbereichs (46) in einem Substanzbehälter (5) und einen zu dem Mundstück (6) führenden Austragkanal (75) für Substanz (48) aufweist, wobei weiter zwei Einstecheinrichtungen (60) vorgesehen sind, mit bevorzugt zwei voneinander gesonderten Halteteilen (62), die jeweils ein Einstechmittel (63) aufweisen und mit denen in sich kreuzenden oder entgegengesetzten Einstechrichtungen (b, c) der Substanzbehälter (5) öffenbar ist, **dadurch gekennzeichnet, dass** die Vorrichtung (1) eine verschwenkbare Verschlusskappe (9) aufweist und dass mit Verschwenken der Verschlusskappe (9) zugleich auf beide Einstecheinrichtungen (60) eingewirkt wird und dass am Ende einer Verschwenkbewegung der Verschlusskappe aus einer Vorrichtungs-Verschlussstellung in eine Vorrichtungs-Offenstellung jede Einstecheinrichtung wieder aus dem Substanzbehälter herausgefahren ist.

2. Vorrichtung (1) nach Anspruch 1, **dadurch gekennzeichnet, dass** die Bewegung beider Einstecheinrichtungen synchron erfolgt, zufolge der Schwenkverlagerung der Verschlusskappe.

3. Vorrichtung (1) nach einem der vorhergehenden Ansprüche, **dadurch gekennzeichnet, dass** unterseitig eines Verschlusskappen-Oberteils (10) und eines Verschlusskappen-Unterteils (11), jeweils gerichtet in Richtung auf eine jeweils zugewandte Vertiefung (21) bzw. (61) ein Nocken (80) angeformt ist, der im Zuge der Schwenkbewegung der Verschlusskappe (9) die Einstechmittel (63) gegen die Rückstellkraft einer Kunststofffeder (65) in Einstechrichtung (b, c) niederdrückt.

4. Vorrichtung (1) nach Anspruch 3, **dadurch gekennzeichnet, dass** die Nocken (80) die Einstecheinrichtung (60) im Bereich des jeweiligen Halteteils (62) beaufschlagen.

5. Vorrichtung (1) nach einem der Ansprüche 3 oder 4, **dadurch gekennzeichnet, dass** zum Ende der Verschwenkbewegung der Verschlusskappe (9) die Nocken (80) den Einflussbereich auf die Einstecheinrichtungen (60) verlassen, welche sich zufolge der Rückstellkraft der Kunststofffedern (65) wieder zurück in ihre Ausgangsstellung bewegen, wobei die Einstechmittel (63) bzw. Einstechbereiche (104) wieder aus den Teilbereichen (46) des Substanzbehälters (5) herausfahren, zur entsprechenden Freigabe der Öffnungen (78, 79) bzw. zur strömungsmäßigen Verbindung dieser Öffnungen mit den Ansaug- und Austragskanälen (74, 75) und/oder, bevorzugt, dass ein Einstechmittel (63) zwei voneinander gesonderte Einstechbereiche (104) aufweist.

6. Vorrichtung (1) nach Anspruch 5, **dadurch gekennzeichnet, dass** zwischen den Einstechbereichen (104) ein sich in Transportrichtung (r) der Substanzbehälter (5) ersteckender Freiraum (64) gegeben ist.

7. Vorrichtung (1) nach einem der Ansprüche 5 oder 6, **dadurch gekennzeichnet, dass** an einem Einstechbereich (104) zugeordnet dem Substanzbehälter (5) zwei oder mehr Einstechspitzen (105) ausgebildet sind.

8. Vorrichtung (1) nach Anspruch 7, **dadurch gekennzeichnet, dass** eine Einstechspitze (105) dornartig ausgebildet ist, mit einem an einem zylindrischen Bereich (106) anschließenden Spitzenbereich (107).

9. Vorrichtung (1) nach einem der Ansprüche,3 bis 8, **dadurch gekennzeichnet, dass** das Halteteil (62) kombiniert mit einer Kunststofffeder (65) ausgebildet ist, wobei, bevorzugt, die Kunststofffeder (65) zwei entgegengesetzt gerichtete Federarme (66) ausbildet, wobei, weiter bevorzugt, die Federarme (66) zugleich zur Führung des Halteteils (62) bei einem Einstechvorgang dienen, und/oder, bevorzugt, die Federarme (66) ausgehend von ihren freien Enden eine Führungsausnehmung (67) aufweisen, die mit einem gehäusefesten Zapfen (68) zusammenwirkt.

10. Vorrichtung (1) nach Anspruch 9, **dadurch gekennzeichnet, dass** die Federarme (66) in einer Seitenansicht, in der sich eine Einstechrichtung (b, c) linienartig darstellt und beide Federarme (66) sich in ihrer Längserstreckung (L) abbilden, konkav gebildet sind, mit dem Halteteil (62) zugeordnet einer Längsmitte der zusammengefassten Federarme (66).

11. Vorrichtung (1) nach einem der vorhergehenden Ansprüche, **dadurch gekennzeichnet, dass** zwei Austragskanäle (75) vorgesehen sind.

12. Vorrichtung (1) nach Anspruch 11, **dadurch gekennzeichnet, dass** die Ansaugkanäle (74) in einer Strömungsrichtung (s) vor dem Mundstück (6) zusammengeführt sind.

13. Vorrichtung (1) nach Anspruch 12, **dadurch gekennzeichnet, dass** ein Ansaugkanal (74) in einer Strömungsrichtung (s) vor dem Substanzbehälter (5) über einen Bypass (103) mit dem Austragskanal (75) verbunden ist.

14. Vorrichtung (1) nach Anspruch 13, **dadurch gekennzeichnet, dass** beide Ansaugkanäle (74) in der Strömungsrichtung (s) vor dem Substanzbehälter (5) über einen Bypass (103) mit dem Austragskanal (75) verbunden sind.

15. Vorrichtung (1) nach einem der vorhergehenden Ansprüche, **dadurch gekennzeichnet, dass** in der Vorrichtung (1) eine Mehrzahl von Substanzbehältern (5) gleichzeitig aufnehmbar sind.

## Claims

1. A Device (1) for inhaling powder-type substances (48), wherein the device (1) has a mouthpiece (6), an insertion mechanism (60) comprising a retaining part (62) having an insertion means (63) for opening a sub-region (46) in a substance container (5) containing substance (48,48'), and a discharge channel (75) for substance (48) leading to the mouthpiece (6), wherein two insertion mechanisms (60) are further provided, comprising preferably two retaining parts (62), which are separate from one another, which each have an insertion means (63), and by means of which the substance container (5) can be opened in intersecting or opposing insertion directions (b, c), **characterised in that** the device (1) has a pivotable closure cap (9) and that both insertion mechanisms (60) are acted on simultaneously with the pivoting of the closure cap (9), and that each insertion mechanism is moved out of the substance container again at the end of a pivoting movement of the closure cap from a device closed position into a device open position.

2. Device (1) according to claim 1, **characterised in that** the movement of both insertion mechanisms takes place synchronously, according to the pivoting displacement of the closure cap.

3. Device (1) according to one of the preceding claims, **characterised in that** a cam (80) is formed on the underside of a closure cap upper part (10) and a closure cap bottom part (11), in each case directed in the direction of a respective facing depression (21) or (61), which cam presses down the insertion means (63) against the restoring force of a plastic spring (65) in the piercing direction (b, c) in the course of the pivoting movement of the closure cap (9).

4. Device (1) according to claim 3, **characterised in that** the cams (80) act on the insertion mechanism (60) in the region of the respective retaining part (62).

5. Device (1) according to one of claims 3 or 4, **characterised in that** at the end of the pivoting movement of the closure cap (9) the cams (80) leave the area of influence on the insertion mechanism (60), which move back into their initial position due to the restoring force of the plastic springs (65), the insertion means (63) or insertion region (104) moving out of the subregions (46) of the substance container (5) again for the corresponding release of the openings (78, 79) or for the flow connection of these openings with the substance container (5) the insertion means (63) or insertion regions (104) move out of the sub-region (46) of the substance container (5) again, for the corresponding release of the openings (78, 79) or for the flow connection of these openings with the suction and discharge channels (74, 75) and/or, preferably, that a insertion means (63) has two insertion regions (104) separate from one another.

6. Device (1) according to claim 5, **characterised in that** a free space (64) extending in the transport direction (r) of the substance containers (5) is provided between the insertion regions (104).

7. Device (1) according to one of claims 5 or 6, **characterised in that** two or more insertion tips (105) are formed on an insertion region (104) associated with the substance container (5).

8. Device (1) according to claim 7, **characterised in that** an insertion tip (105) is of mandrel-like design, with a tip region (107) adjoining a cylindrical region (106).

9. Device (1) according to one of the claims 3 to 8, **characterised in that** the retaining part (62) is formed in combination with a plastic spring (65), wherein, preferably, the plastic spring (65) forms two oppositely directed spring arms (66), wherein, further preferably, the spring arms (66) serve at the same time to guide the retaining part (62) during a piercing process, and/or, preferably, the spring arms (66) have, starting from their free ends, a guide recess (67) which cooperates with a journal (68) fixed to the housing.

10. The device (1) according to claim 9, **characterised in that** the spring arms (66) are formed concave in a side view, in which an insertion direction (b, c) is line-like and both spring arms (66) are represented in their longitudinal extension (L), with the retaining part (62) associated with a longitudinal centre of the combined spring arms (66).

11. Device (1) according to one of the preceding claims, **characterised in that** two discharge channels (75) are provided.

12. Device (1) according to claim 11, **characterised in that** the suction channels (74) are brought together in a flow direction (s) in front of the mouthpiece (6).

13. Device (1) according to claim 12, **characterised in that** a suction channel (74) is connected to the discharge channel (75) in a flow direction (s) upstream of the substance container (5) via a bypass (103).

14. Device (1) according to claim 13, **characterised in that** both suction channels (74) are connected to the discharge channel (75) in the direction of flow (s) upstream of the substance container (5) via a bypass (103).

15. Device (1) according to one of the preceding claims, **characterised in that** a plurality of substance containers (5) can be accommodated simultaneously in the device (1).

## Revendications

1. Dispositif (1) pour l'inhalation de substances pulvérulentes (48), le dispositif (1) présentant un embout buccal (6), un dispositif de perçage (60) avec une partie de retenue (62) présentant un moyen de perçage (63) pour l'ouverture d'une zone partielle (46) contenant une substance (48, 48') dans un récipient de substance (5) et un canal de sortie (75) menant à l'embout buccal (6) pour la substance (48), deux dispositifs de perçage (60) étant en outre prévus, avec de préférence deux parties de retenue (62) séparées l'une de l'autre, qui présentent chacune un moyen de perçage (63) et avec lesquelles le récipient de substance (5) peut être ouvert dans des directions de perçage (b, c) qui se croisent ou qui sont opposées, **caractérisées, en ce que** le dispositif (1) présente un capuchon de fermeture (9) pivotant et **en ce que**, avec le pivotement du capuchon de fermeture (9), on agit simultanément sur les deux dispositifs de perçage (60) et **en ce que**, à la fin d'un mouvement de pivotement du capuchon de fermeture d'une position de fermeture du dispositif dans une position d'ouverture du dispositif, chaque dispositif de perçage est à nouveau sorti du récipient de substance.

2. Dispositif (1) selon la revendication 1, **caractérisé en ce que** le mouvement des deux dispositifs d'insertion est synchrone, suite au déplacement pivotant du capuchon de fermeture.

3. Dispositif (1) selon l'une des revendications précédentes, **caractérisé en ce qu'**une came (80) est formée sur la face inférieure d'une partie supérieure de capuchon (10) et d'une partie inférieure de capuchon (11), respectivement orientées en direction d'un renfoncement (21) et d'un renfoncement (61) respectivement tournés vers elles, qui, au cours du mouvement de pivotement du capuchon (9), enfonce les moyens d'enfoncement (63) dans le sens d'enfoncement (b, c) à l'encontre de la force de rappel d'un ressort en plastique (65).

4. Dispositif (1) selon la revendication 3, **caractérisé en ce que** les cames (80) sollicitent le dispositif d'insertion (60) dans la zone de la pièce de retenue (62) respective.

5. Dispositif (1) selon l'une des revendications 3 ou 4, **caractérisé en ce que**, à la fin du mouvement de pivotement du capuchon (9), les cames (80) quittent la zone d'influence sur les dispositifs de perçage (60), qui reviennent dans leur position de départ sous l'effet de la force de rappel des ressorts en matière plastique (65), les moyens de perçage (63) ou les dispositifs de perçage (60) étant alors déplacés vers la position de fermeture (104) sortent à nouveau des zones partielles (46) du réservoir de substance (5), pour libérer de manière correspondante les ouvertures (78, 79) ou pour relier ces ouvertures aux canaux d'aspiration et de décharge (74, 75) et/ou, de préférence, qu'un moyen de perçage (63) présente deux zones de perçage (104) séparées l'une de l'autre.

6. Dispositif (1) selon la revendication 5, **caractérisé en ce qu'**un espace libre (64) s'étendant dans la direction de transport (r) des récipients de substance (5) est donné entre les zones de perçage (104).

7. Dispositif (1) selon l'une des revendications 5 ou 6, **caractérisé en ce que** deux ou plusieurs pointes de perçage (105) sont formées sur une zone de perçage (104) associée au récipient de substance (5).

8. Dispositif (1) selon la revendication 7, **caractérisé en ce qu'**une pointe de perçage (105) est réalisée sous forme de mandrin, avec une zone de pointe (107) se raccordant à une zone cylindrique (106).

9. Dispositif (1) selon l'une des revendications 3 à 8, **caractérisé en ce que** la pièce de retenue (62) est réalisée en combinaison avec un ressort en matière plastique (65), de préférence le ressort en matière plastique (65) formant deux bras de ressort (66) orientés en sens inverse, de préférence encore les bras de ressort (66) servant en même temps au guidage de la pièce de retenue (62) lors d'une opération de perçage, et/ ou de préférence les bras de ressort (66) présentant, à partir de leurs extrémités libres, un évidement de guidage (67) qui coopère avec un tenon (68) solidaire du boîtier.

10. Dispositif (1) selon la revendication 9, **caractérisé en ce que** les bras élastiques (66) sont formés de manière concave dans une vue latérale, dans laquelle une direction d'enfoncement (b, c) se présente sous forme de ligne et les deux bras élastiques (66) se représentent dans leur extension longitudinale (L), avec la partie de retenue (62) associée à un centre longitudinal des bras élastiques (66) réunis.

11. Dispositif (1) selon l'une des revendications précédentes, **caractérisé en ce qu'**il est prévu deux canaux d'évacuation (75).

12. Dispositif (1) selon la revendication 11, **caractérisé en ce que** les canaux d'aspiration (74) sont réunis dans une direction d'écoulement (s) en amont de l'embouchure (6).

13. Dispositif (1) selon la revendication 12, **caractérisé en ce qu'**un canal d'aspiration (74) est relié au canal de décharge (75) dans un sens d'écoulement (s) en amont du réservoir de substance (5) par une dérivation (103).

14. Dispositif (1) selon la revendication 13, **caractérisé en ce que** les deux canaux d'aspiration (74) sont reliés au canal d'évacuation (75) dans le sens d'écoulement (s) en amont du réservoir de substance (5) par une dérivation (103).

15. Dispositif (1) selon l'une des revendications précédentes, **caractérisé en ce qu'**une pluralité de récipients de substance (5) peuvent être reçus simultanément dans le dispositif (1).
